# EUROPEAN PATENT SPECIFICATION

(11) **EP 0 652 948 B1**
(45) Date of publication and mention of the grant of the patent: **14.11.2001**
(21) Application number: 93910867.6
(22) Date of filing: 28.04.1993
(51) Int. Cl.: C12N 15/00, C12N 5/10, A61K 49/00, A61K 35/00, A61K 31/00, C12N 15/85, A01K 67/027, A61K 48/00, C07K 14/025, C07K 14/495, C07K 14/82

(54) **DEVELOPMENT OF A VECTOR TO TARGET GENE EXPRESSION TO THE EPIDERMIS OF TRANSGENIC ANIMALS**
ENTWICKLUNG EINES VEKTORS ZUR ERREICHUNG VON GENEXPRESSION IN DER EPIDERMIS TRANSGENER TIERE
CREATION D'UN VECTEUR APPELE A CIBLER UNE EXPRESSION GENIQUE DANS L'EPIDERME D'ANIMAUX TRANSGENIQUES

(30) Priority: 30.04.1992 US 876289
(43) Date of publication of application: 17.05.1995
(73) Proprietor: BAYLOR COLLEGE OF MEDICINE, Houston, TX 77030 (US); THE UNITED STATES OF AMERICA, as represented by the Secretary of the Department of Health and Human Services, Bethesda, Maryland 20892 (US)
(72) Inventor: ROOP, Dennis R., Houston, TX 77030 (US); ROTHNAGEL, Joseph A., Houston, TX 77096 (US); GREENHALGH, David A., Houston, TX 77025 (US); YUSPA, Stuart H., Bethesda, MD 20817 (US)
(74) Representative: Goldin, Douglas Michael
(86) International application number: US9303985
(87) International publication number: WO9322430

(56) References cited:
- US-A- 4 497 796
- US-A- 4 736 866
- THE JOURNAL OF INVESTIGATIVE DERMATOLOGY, vol. 95, no. 5, November 1990, pages 59s-61s, XP000612194 ROTHNAGEL, J.A. ET AL.: "Targeting gene expression to the epidermis of transgenic mice: potential applications to genetic skin disorders"
- CLINICAL RESEARCH, vol. 39, no. 2, 1 May 1991, page 259a XP000612924 ROTHNAGEL, J.A. ET AL.: "Use of transgenic mice to identify regulatory sequences 5' to the human K1 keratin gene"
- CLINICAL RESEARCH, vol. 39, no. 4, 29 January 1992, page 850a XP000612925 HENDRIX, J.A. ET AL.: "Differences in fetal expression of two human K1 keratin gene fragments in transgenic mice"
- CELL, vol. 62, 24 August 1990, NA US, pages 697-708, XP002021485 BAILLEUL, B. ET AL.: "Skin hyperkeratosis and papilloma formation in transgenic mice expressing a ras oncogene from a suprabasal keratin promoter"
- Meeting of the Society of Investigative Dermatology, Baltimore, USA, APRIL 29- MAY 2 1992 XP002021486 & CLINICAL RESEARCH , vol. 40, no. 2, 1992, page 546a XP000612751 GREENHALGH, D.A. ET AL.: "Targeting v-fos expression to the epidermis of transgenic mice and cooperation with targeted v-ras-Ha oncogene in epidermal carcinogenesis"
- MOLECULAR CARCINOGENESIS, vol. 7, no. 2, 1993, pages 99-110, XP000612740 GREENHALGH, D.A. ET AL.: "Induction of epidermal hyperplasia, hyperkeratosis, and papillomas in transgenic mice by a targeted v-Ha-ras oncogene"
- ONCOGENE, vol. 8, no. 8, August 1993, pages 2145-2157, XP000612775 GREENHALGH, D.A. ET AL.: "Hyperplasia, hyperkeratosis and benign tumor production in transgenic mice by a targeted v-fos oncogene suggest a role for fos in epidermal differentiation and neoplasia"
- Cell Growth & Differentiation, Volume 2, issued February 1991, ROSENTHAL et al., "A Human Epidermal Differentiation-Specific Keratin Gene is Regulated by Calcium but not Negative Modulators of Differentiation in Transgenic Mouse Keratinocytes", pages 107-113, see the entire document.
- Proc. Natl. Acad. Sci. USA, Volume 82, issued April 1985, JOHNSON et al., "Structure of a Gene for the Human Epidermal 67-kDa Keratin", pages 1896-1900, see the entire document.
- Science, Volume 217, issued 03 September 1982, DHAR et al., "Nucleotide Sequence of the p21 Transforming Protein of Harvey Murine Sarcoma Virus", pages 934-937, see the entire document.
- Virology, Volume 145, issued 1985, SEEDORF et al., "Human Papillomavirus Type 16 DNA Sequence", pages 181-185, see the entire document.
- Journal of Virology, Volume 61, No. 4, issued April 1987, PIRISI et al., "Transformation of Human Fibroblasts and Keratinocytes with Human Papillomavirus Type 16 DNA", pages 1061-1066, see the entire document.
- Nature, Volume 352, issued 29 August 1991, ACSADI et al., "Human Dystrophin Expression in MDX Mice After Intramuscular Injection of DNA Constructs", pages 815-818, see the entire document.
- Science, Volume 254, issued 06 December 1991, DHAWAN et al., "Systemic Delivery of Human Growth Hormone by Injection of Genetically Engineered Myoblasts", pages 1509-1512, see the entire document.

## Description

This invention was partially supported by grants from the United States government under HD25479, AI30283 and CA52607 awarded by the National Institutes of Health. Further, this work was partially performed at the National Institutes of Health in the Laboratory of Cellular Carcinogenesis and Tumor Promotion, Division of Cancer Etiology, National Cancer Institute. The government has certain rights in the invention.

### FIELD OF THE INVENTION

The present invention relates to expression vectors for use in expressing polypeptides in epidermal cells of transgenic animals. More particularly it relates to vectors containing the K1 keratin gene promoter, its 5' flanking region, its 5' transcribed but untranslated region, its first intron and intron/exon boundary, its 3' transcribed but untranslated region, its contiguous non-coding DNA containing the gene's natural transcriptional termination region and its 3' flanking region.

### BACKGROUND OF THE INVENTION

The ability to stably introduce genes into the germline of mice has greatly enhanced prospects for the generation of animal models of human diseases (Palmiter and Brinster, Ann. Rev. Genet., Vol. 20, pp. 465-499 (1986)). The need for such animal models is becoming increasingly apparent as novel pharmaceuticals are developed which are specifically designed to inhibit expression of human viruses or counteract the effect of mutated genes that occur in human diseases. Current efficacy assessments of these new therapeutic agents are restricted to *in vitro* models which do not allow evaluation of delivery routes nor assessment of other factors known to affect disease processes *in vivo*, such as blood supply, an intact immune system, humoral and cell-mediated growth controls and physical barriers to disease progression. In addition, the prospects for utilizing gene therapy to treat human disorders are coming closer to reality. Therefore, animal models of human diseases would be useful to assess the therapeutic potential of these approaches. The epidermis is an attractive tissue for the development animal models since it serves as a general model for other squamous epithelia and its accessibility allows macroscopic observation of pathological events and easy assessment of therapeutic potential. The development of a vector which specifically targets gene expression to the epidermis of transgenic animals is the subject of this invention.

The epidermis is a continuously regenerating stratified squamous epithelium. Differentiated epidermal cells are the progeny of proliferative cells located in the basal cell layer and there is substantial evidence suggesting that the regeneration process occurs in proliferative units composed of slowly cycling, self-renewing stem cells, proliferative but non-renewing transit amplifying cells, and post-mitotic maturing epidermal cells (Iversen, et al., Cell Tissue Kinet., Vol. 1, pp. 351-367, (1968); MacKenzie, et al., Nature, Vol. 226, pp. 653-655, (1970); Christophers, et al., J. Invest. Dermatol., Vol. 56, pp. 165-170, (1971); Potten, In Stem Cells: Their Identification and Characterization, pp. 200-232, (1983); Cotsarelis, et al., Cell, Vol. 61, pp. 1329-1337, (1990)). The maturation process (terminal differentiation) is initiated when epidermal cells withdraw from the cell cycle and migrate from the basal layer into the spinous layer. Maturation continues as spinous cells migrate into the granular layer and terminates with the formation of the stratum corneum. Morphological and biochemical studies have shown that terminal differentiation occurs in stages. (Matoltsy, J. Invest. Dermatol., Vol. 65, pp. 127-142, (1975)). Keratins K5 and K14 are major products of basal epidermal cells (Woodcock-Mitchell, et al., J. Cell Biol., Vol. 95, pp. 580-588, (1982)). These proteins assemble into 10 nm filaments (intermediate filaments [IF]) and, together with microtubules (tubulin) and microfilaments (actin), comprise the cytoskeleton of epidermal cells (Steinert, P.M., et al., Cell, Vol. 42, pp. 411-419, (1985)). One of the earliest changes associated with the commitment to differentiation and migration into the spinous layer is the induction of another differentiation-specific pair of keratins (K1 and K10). IF containing K1 and K10 replace those containing K5 and K14 as the major products of cells in the spinous layer (Woodcock-Mitchell, et al., J. Cell Biol., Vol. 95, pp. 580-588, (1982); Roop, et al., Proc. Natl. Acad. Sci., USA, Vol. 80, pp. 716-720, (1983); Schweizer, et al, Cell, Vol. 37, pp. 159-170, (1984)). The keratin IF formed by these proteins assemble into bundles. In the granular layer, another high molecular weight non-IF protein is synthesized, which is processed into filaggrin, and is thought to promote keratin filament aggregation and disulfide-bond formation (Dale, B.A., et al., Nature, Vol. 276, pp. 729-731, (1978); Harding, C.R., et al., J. Mol. Biol., Vol. 170, pp. 651-673, (1983)). In the final stage of epidermal cell maturation, transglutaminase catalyzes the crosslinking of involucrin and loricrin, by the formation of (γ-glutamyl) lysine isopeptides, into a highly insoluble cornified envelope which is located just beneath the plasma membrane (Rice and Green, Cell Vol. II, pp. 417-422 (1977) Mehrel, et al., Cell, Vol. 61, pp. 1103-1112, (1990)).

Genes or cDNAs encoding the major keratins expressed in epidermal cells have now been cloned: K5 (Lersch, et al., Mol. and Cell Biol., Vol. 8, pp. 486-493, (1988), K14 (Marchuk, et al., Proc. Natl. Acad. Sci, USA, Vol. 82, pp. 1609-1613, (1985); Knapp, et al., J. Biol. Chem, Vol. 262, pp. 938-945, (1987); Roop, et al., Cancer Res., Vol. 48, pp. 3245-3252, (1988), K1 (Steinert, et al., J. Biol. Chem., Vol. 260, pp. 7142-7149, (1985) and K10 (Krieg, et al., J. Biol. Chem., Vol. 260, pp. 5867-5870, (1985)). Northern blot analysis and *in situ* hybridization studies suggest that keratin genes K5 and K14 are predominantly transcribed in the proliferating basal layer and transcription of keratin genes K1 and K10 is induced as cells migrate into the spinous layer (Lersch, et al., Mol. and Cell Biol., Vol. 8, pp. 486-493, (1988); Knapp, et al., J. Biol. Chem., Vol. 262, pp. 938-945, (1987); Roop, et al., Cancer Res., Vol. 48, pp. 3245-3252, (1988)). Genes encoding rat (Haydock, et al., J. Biol. Chem., Vol. 261, pp. 12520-12525, (1986)) and mouse (Rothnagel, et al., J. Biol. Chem., Vol. 262, pp. 15643-15648, (1987)) filaggrin have now been identified and *in situ* hybridization experiments have confirmed that transcription of this gene is restricted to the granular layer (Rothnagel, et al, J. Biol. Chem., Vol. 262, pp. 15643-15648, (1987); Fisher, et al. J. Invest. Dermatol., Vol. 88, pp. 661-664, (1987)). To date, loricrin is the only gene encoding a component of the cornified envelope to be studied at the molecular level by *in situ* hybridization and transcripts of this gene are restricted to the granular layer (Mehrel, et al., Cell, Vol. 61, pp. 1103-1112, (1990)).

From this description of gene expression in the epidermis, there would appear to be many candidate genes from which to choose for targeting to the epidermis. However, this is not the case. Keratins K5 and K14, expressed in the proliferative compartment of the epidermis, are not only expressed in the epidermis but in all squamous epithelia. Furthermore, these genes are expressed early in development (Dale and Holbrook, In: Current Topics in Developmental Biology, pp. 127-151, (1987)) and this could cause lethality *in utero.* The generation of animal models of hyperproliferative diseases such as cancer and psoriasis would most likely require expression in the basal compartment in cells with proliferative potential, therefore, genes expressed post-mitotically such as keratins K1 and K10 and those encoding filaggrin and components of the cell envelope (involucrin and loricrin) would be excluded on this basis.

Cell Growth and Differentiation, 1991 vol 2 p107-113 describes expression of human and mouse K1 genes including the regulatory elements which respond to Ca²⁺ ions. Clin Research 40(2) 1992 and J. Invest. Dermat 95 No 5 1990 59-61 suggest that the K1 keratin gene may be used to generate a targeting vector for expression of desired proteins in the epidermis but do not identify the specific regulatory sequences that are required.

The present invention, however, demonstrates that a 12kb fragment of the human keratin gene K1 (HK1) contains sequences regulating tissue and developmental specific expression in transgenic mice. This fragment lacks sequences responsive to negative control of differentiation specific expression resulting in expression of the HK1 gene in some cells of the basal cell compartment of the epidermis. Although regulatory elements of the HK1 gene fail to completely mimic the expression pattern of the endogenous mouse K1 gene, they are ideally suited for targeting gene expression for the following reasons: (1) expression only occurs in the epidermis and not other squamous epithelia; (2) expression occurs at a late stage of development (day 15) and, therefore, is unlikely to result in lethality *it utero*; (3) expression occurs in a large proportion of basal cells that have proliferative potential.

### SUMMARY OF THE INVENTION

An object of the present invention is a keratin K1 vector for expressing nucleic acid sequences in the epidermis.

An additional object of the present invention is a keratin K1 vector containing an oncogene.

A further object of the present invention is a bioreactor for producing proteins, polypeptides and antisense RNA in transduced epidermal cells.

An additional object of the present invention is an *in vivo* method of transducing epidermal cells with a keratin K1 vector.

A further object of the present invention is provision of a transgenic animal containing the keratin K1 epidermal vector.

An additional object of the present invention is the provision of a transgenic animal for the study of cancer.

Another object of the present invention is a method of treating skin ulcers.

A further object of the present invention is an enhanced method of wound healing or healing of surgical incisions.

An additional object of the present invention is a method of treating psoriasis.

An additional object of the present invention is a method of treating skin cancer.

An additional object of the present invention is a vaccination procedure using the keratin K1 epidermal vector.

Thus, in accomplishing the foregoing objects, there is provided in accordance with one aspect of the present invention a keratin K1 vector for expression of a nucleic acid cassette in the epidermis comprising: a 5' flanking region of the keratin K1 gene, said 5' flanking sequence including a keratin K1 promoter, a 5' transcribed but untranslated region and a first intron all in sequential and positional relationship for expression of a nucleic acid cassette; a 3' flanking region of the keratin K1 gene containing Vitamin D₃ regulatory sequences, a 3' transcribed but untranslated region and contiguous noncoding DNA containing a transcriptional termination region; and a polylinker having a plurality of restriction endonuclease sites, said polylinker cconnecting the 5' flanking region to the 3' flanking region and said polylinker further providing a position for insertion of the nucleic acid cassette.

In specific embodiments of the present invention the keratin K1 vector has a 5' flanking region of approximately 1.2 kb and a 3' flanking region of approximately 3.9 kb. The 5' flanking region may include an intron/exon boundary.

In alternative embodiments of the present invention there is a further addition of approximately 8.0 kb of 5' flanking sequence from the 18 kb Eco RV fragment onto the end of the vector.

In another alternative embodiment the Vitamin D₃ regulatory element within the human K1 keratin gene is identified and utilized to suppress expression of the keratin K1 vector.

In the specific embodiments of the present invention the keratin K1 vector is used to transduce epidermal cells to form bioreactors. The bioreactors produce a variety of proteins, polypeptides and RNAs. Additionally, the vector can be used to form transgenic animals. The transgenic animals can be used to study cancer, drug reactions and treatments.

The keratin K1 vector can also be used for the treatment of a variety of diseases, including wounds, surgical incisions, psoriasis, skin ulcers and skin cancer and can be used for the production of vaccines.

Other and further objects, features and advantages will be apparent from the following description of the presently preferred embodiments of the invention which are given for the purposes of disclosure when taken in conjunction with the accompanying drawings.

### BRIEF DESCRIPTION OF THE DRAWINGS

**Figure 1** is a schematic drawing of the human keratin K1 gene (HK1) and the expression vector derived from its regulatory sequences.
**Figure 2** shows the expression characteristics of the HK1 vector *in vivo* in transgenic mice utilizing a reporter gene encoding E. coli β-galactosidase.
**Figure 3** demonstrates the suppression of the SV40 promoter by a novel negative regulatory element from the HK1 gene (HK1.NRE) in the presence of Vitamin D₃.
**Figure 4** is a schematic drawing of the HK1 vector containing the coding sequence of v-ras^{Ha} protein of Harvey Murine Sarcoma Virus.
**Figure 5** is a schematic drawing of the HK1 vector containing the coding sequence of the v-fos protein from a FBJ/FBR chimeric plasmid.
**Figure 6** is a schematic drawing of the HK1 expression vector containing the coding sequences of the E6 and E7 proteins from human papilloma virus 18.
**Figure 7** is a schematic drawing of the HK1 vector containing the coding sequence of TGF-α.
**Figure 8** is a schematic drawing of the HK1 vector containing the coding sequence of the trans-regulatory protein *tat,* from human immunodeficiency virus.
**Figure 9** is a schematic drawing of an 18kb Eco RV fragment containing the HK1 gene.
**Figure 10** is a schematic drawing of a derivative of the HK1 vector containing additional 5' flanking sequences which restrict expression to differentiated epidermal cells.

The drawings are not necessarily to scale, and certain features of the invention may be exaggerated in scale and shown in schematic form in the interest of clarity and conciseness.

### DETAILED DESCRIPTION OF THE INVENTION

It will be readily apparent to one skilled in the art that varying substitutions and modifications may be made to the invention disclosed herein without departing from the scope and spirit of the invention.

The term "transformed" as used herein refers to the process or mechanism of inducing changes in the characteristics (expressed phenotype) of a cell by the mechanism of gene transfer whereby DNA is introduced into a cell in a form where it expresses a specific gene product or alters expression of endogenous gene products.

The term "transduction" as used herein refers to the process of introducing a DNA expression vector into a cell. Various methods of transduction are possible, including microinjection, CaPO₄, lipofection (lysosome fusion), use of a gene gun and DNA vector transporter. The human keratin K1 vector can be transduced into the epidermal cells by any of the variety of ways described above.

The term "DNA vector transporter" as used herein refers to those molecules which bind to DNA vectors and are capable of being taken up by epidermal cells. DNA transporter is a molecular complex capable of non-covalent binding to DNA and efficiently transporting the DNA through the cell membrane. Although not necessary, it is preferable that the transporter also transport the DNA through the nuclear membrane.

The term "transient" as used in transient transfection, transient transduction or transiently transformed relates to the introduction of genes into the epidermal cells to express specific proteins, polypeptides and RNA wherein the introduced genes are not integrated into the host cell genome and accordingly are eliminated from the cell over a period of time. Transient expression relates to the expression of gene products during the period of transient transfection. Additionally, transient can refer to a stable transfection or transduction into cells, where the cells die and are sloughed off from the skin. Thus, the transformed cells are only transiently available for the expression of the incorporated genes.
The term "stable" as used in stable transfection, stable transduction or stably transformed refers to the introduction of genes into the chromosome of the targeted cell where it integrates and becomes a permanent component of the genetic material in that cell. Gene expression after stable transduction can permanently alter the characteristics of the cell leading to stable transformation. An episomal transformation is a variant of stable transformation in which the introduced gene is not incorporated into the host cell chromosomes but rather is replicated as an extrachromosomal element. This can lead to apparently stable transformation of the characteristics of the cell. As indicated above, in epidermal cells, which are sloughed from the body through the skin, stable transformation can become transient transformation because the cells are lost.

The term "nucleic acid cassette" as used herein refers to the genetic material of interest which can express a protein, polypeptide or RNA and which is capable of being incorporated into the epidermal cells. The nucleic acid cassette is positionally and sequentially oriented within the keratin K1 vector such that the nucleic acid in the cassette can be transcribed into RNA or antisense RNA and, when necessary, translated into proteins or polypeptides in the transformed epidermal cells. A variety of proteins and polypeptides can be expressed in the transformed epidermal cells by the sequence in the nucleic acid cassette. These proteins or polypeptides which can be expressed include hormones, growth factors, enzymes, clotting factors, apolipoproteins, receptors, drugs, tumor antigens, viral antigens, parasitic antigens, bacterial antigens and oncogenes. Specific examples of these compounds include proinsulin, insulin, growth hormone, insulin-like growth factor I, insulin-like growth factor II, insulin growth factor binding protein, epidermal growth factor TGF-α, dermal growth factor PDGF, angiogenesis factor, for instance, acid fibroblast and basic fibroblast growth factors and angiogenin, matrix protein, such as, Type IV collagen, Type VII collagen, laminin, nidogen and proteins from viral, bacterial and parasitic organisms which can be used to induce an immunologic response.

In addition, the nucleic acid cassette can encode a "transforming gene" which encompasses viral oncogenes, endogenous proto-oncogenes and activated proto-oncogenes. A variety of oncogenes are known in the art. The term "oncogene" means those genes which cause cancer and include both viral and cellular oncogenes, many of which are homologous to DNA sequences endogenous to rodents and/or humans. The term oncogene includes both the viral sequence and the homologous endogenous sequences. Some examples of transforming genes are listed in Table 1.

**Table 1.**

| Transforming Genes | |
|---|---|
| ABBREVIATION | NAME |
| Ha-*ras* | Harvey Murine Sarcoma Virus |
| Ki-*ras* | Kirsten Murine Sarcoma Virus |
| N-*ras* | Neuroblastoma oncogene |
| *fos* | FBJ or FBR osteosarcoma virus |
| *myc* | Avian MC29 myelocytomatosis virus |
| *src* | Rous sarcoma virus |
| *sis* | Simian sarcoma virus/PDGF β chain |
| *erb*A | Avian erythroblastosis virus/Thyroxine T3 receptor |
| *erb*B | Avian erythroblastosis virus/Truncated EGF receptor |
| *jun* | Avian sarcoma virus 17 |
| p Large T | Polyomavirus transforming gene |
| p Middle T | Polyomavirus transforming gene |
| HPV E7 | Early region transforming gene from human papillomavirus 6, 11, 16, 18 |
| HPV E6 | Early region transforming gene from human papilloma virus 6, 11, 16, 18 |
| HPV E5 | Early region transforming gene from human papilloma virus 6, 11, 16, 18 |
| *tat* | HIV transforming gene |
| EIA | Adenovirus early region 1A |
| Rb | Mutated retinoblastoma gene |
| p53 | Mutated p53 anti-oncogene |
| WT1 | Mutated Wilms tumor anti-oncogene |
| TGF-α | Transforming growth factor α |
| TGF-β | Transforming growth factor β |
| EGFR | Mutated epidermal growth factor receptor |
| RAR | Mutated retinoic acid receptor |
| VD₃R | Mutated vitamin D₃ receptor |
| PKC | Mutated protein kinase C |

The genetic material which is incorporated into the epidermal cells using the keratin K1 vector includes DNA not normally found in epidermal cells, DNA which is normally found in epidermal cells but not expressed at physiologically significant levels, DNA normally found in epidermal cells and normally expressed at physiological desired levels, any other DNA which can be modified for expression in epidermal cells, and any combination of the above.

The term "keratin K1 vector" or "HK1 vector" as used herein is a vector which is useful for expression of a nucleic acid sequence in epidermal cells. The keratin K1 vector comprises a 5' flanking region of the keratin K1 gene, said flanking region including a promoter, a first intron and an intron/exon boundary all in sequential and positional relationship for the expression of a nucleic acid cassette; a 3' flanking sequence of a keratin K1 gene; and a poly-linker. The poly-linker includes a plurality of restriction endonuclease sites. The polylinker connects the 5' flanking region to the 3' flanking sequence and further provides a position for insertion of the nucleic acid cassette.

The sequence for the 3' flanking region of the human keratin K1 gene contains regulator elements and is used for preparing the keratin K1 vector. It is shown in SEQ. ID No. 1. The keratin K1 vector has a 5' flanking region comprising nucleotides 1 to 1246 of SEQ. ID. No. 1; a 3' flanking sequence containing regulatory sequences comprises nucleotides 6891 to 10747 of SEQ. ID. No. 1; and a poly linker comprising nucleotides 2351 to 2376 of SEQ. ID. No. 2 (the HK1 expression vector).

The keratin K1 vector has a 5' flanking region of approximately 1.2 kb, an intron and intron/exon boundary of approximately 1.0 kb and a 3' flanking sequence of approximately 3.9 kb.

The restriction endonuclease sites found in the linker and poly-linker of the keratin K1 vector can be any restriction endonucleases which will allow insertion of the nucleic acid cassette. In the preferred embodiment they are usually selected from the group consisting of Bam HI, Kpn I, Cla I, Not I, Xma I, and Bgl II.

One skilled in the art will readily recognize that there are a variety of ways to introduce the keratin K1 vector into epidermal cells. The vectors can be inserted either *in vivo* or *ex vivo*. The mode of insertion will, to a certain degree, determine the available methods for the insertion. The *in vivo* insertion is preferred for gene therapy. In this procedure the human keratin K1 vector is contacted with epidermal cells for sufficient time to transform the epidermal cells.

One embodiment of the present invention includes a bioreactor. A bioreactor is comprised of transformed epidermal cells which contain the keratin K1 vector. Once the vector is inserted in the epidermal cells, the epidermal cells will express the nucleic cassette and produce the protein, polypeptide or antisense RNA of interest. This can be done either *in vivo* or *ex vivo*. Any compound which can be encoded in, and expressed by, the nucleic acid cassette can be produced by the bioreactor.

One method for *ex vivo* introduction of the keratin K1 vector into epidermal cells includes a cotransfection of the vector with a selectable marker. The selectable marker is used to select those cells which have become transformed. The cells can then be used in any of the methods described in the present invention.

Another embodiment of the present invention is a method of making transgenic animals comprising the steps of inserting the human keratin K1 vector into the embryo of the animal. The transgenic animal can include the resulting animal in which the vector has been inserted into the embryo or any progeny. The term progeny as used herein includes direct progeny of the transgenic animal as well as any progeny of succeeding progeny. Thus, one skilled in the art will readily recognize that if two different transgenic animals have been made using different genes in the nucleic acid cassette and they are mated, the possibility exists that some of the resulting progeny will contain two or more introduced sequences. One skilled in the art will readily recognize that by controlling the matings, transgenic animals with multiple vectors can be made.

In the transgenic animals that contain the human keratin K1 vector in its germ and somatic cells, the nucleic acid cassette of the said vector is only expressed in the epidermal cells. This is a distinct advantage over other transgenic animal models where there is not as much control over the expression of the sequence in the tissues.

In the preferred embodiment, the transgenic animal will contain an oncogene sequence in the nucleic acid cassette. Preferably the animal is a rodent. The transgenic animal can be used in any method for studying a variety of diseases including the origin of cancer, the treatment of cancer, interaction of the cancer with the environment as well as for looking at drugs, pharmaceuticals and other chemical interactions. The transgenic animals are useful in any assay in which the skin cells of the animal can be used.

One specific embodiment of the present invention is a method for the enhanced healing of a wound or surgical incision. This method comprises the *in vivo* transduction of epidermal cells with a keratin K1 vector. The nucleic acid cassette of said vector contains a nucleic acid sequence for a growth factor.

In the preferred embodiment for the treatment of wounds or surgical incisions, a plurality of vectors are introduced into the epidermal cells. In the plurality of vectors, the cassette of at least one vector contains a nucleic acid sequence for an epidermal growth factor (TGF-α), the cassette of at least one vector contains a dermal growth factor (PDGF), a cassette of at least one vector contains a nucleic acid sequence for a matrix protein to anchor the epidermis to the dermis, and a cassette of at least one vector contains a nucleic acid sequence for an angiogenesis factor. The sequence for matrix proteins can be selected from any sequences useful for the anchoring of the epidermis to the dermis but are usually selected from the group consisting of Type IV collagen, laminin, nidogen, and Type VII collagen. The angiogenesis factor is usually selected from the group consisting of acid fibroblast and basic fibroblast growth factors, and angiogenin. The combination of the vectors provides all of the necessary elements for quick and rapid enhancement of healing of wounds or surgical incisions. This procedure is very helpful in the case of plastic or reconstructive surgery. Furthermore, skin ulcers can be treated by following similar procedures as described for wound healing or surgical incision. These procedures for healing of wounds, surgical incisions and skin ulcers are useful in animals and humans.

In the *ex vivo* approach for treating or healing wounds, surgical incisions and skin lesions, the vectors are first transduced into the epidermal cells *ex vivo*. The transformed epidermal cells are transplanted onto the animal or human to be treated.

Another embodiment of the present invention is a method for treating psoriasis. In this method, epidermal cells are transduced in *in vivo* with a keratin K1 vector. A nucleic acid cassette in said vector contains a nucleic acid sequence for a protein or polypeptide selected from the group consisting of TGF-β, a soluble form of cytokine receptor, and an antisense RNA. The cytokine receptor can be selected from the group consisting of IL-1, IL-6, and IL-8. The antisense RNA sequence is selected from the group consisting of TGF-α, IL-1, IL-6, and IL-8.

In another embodiment of the present invention there is a method of treating skin cancer. This method comprises the steps of *in vivo* transduction of epidermal cells with a keratin K1 vector. The nucleic acid cassette of either vector contains the nucleic acid sequence coding for antisense RNA for the E6 or E7 genes of the human papilloma virus or coding for the normal p53 protein.

It has been found that the keratin K1 vector contains a novel negative regulatory element in its 3' flanking sequence which can be suppressed by Vitamin D₃. With the Vitamin D₃ regulatory element in the vector, the expression of a nucleic acid cassette can be regulated by Vitamin D, a commonly used substance in animals and humans.

The human keratin K1 vector can also be modified by the insertion of additional 5' flanking sequences from an 18 kb Eco RV fragment to its 5' end (nucleotides 6090 to 14180 of SEQ. ID. No. 3). The addition of these sequences allows the human keratin K1 vector to be expressed exactly like the endogenous K1 gene, that is, post mitotically in cells committed to terminal differentiation. Since these cells are programmed to die and will eventually slough into the environment, this is another way of producing transient expression in cells.

An additional embodiment of the present invention is a method for vaccination comprising the step of *in vivo* introduction of a keratin K1 vector into epidermal cells. The nucleic acid cassette in the vectors usually codes for a polypeptide which induces an immunological response. An example of this is the viral capsid from the human papilloma virus. One skilled in the art will readily recognize that any other variety of proteins can be used to generate a immunologic response and thus produce antibodies for vaccination.

The following examples are offered by way of illustration and are not intended to limit the invention in any manner.

### EXAMPLE 1

### Construction and Characterization of a Vector From the HK1 Gene

To target the expression of exogenous DNA to the epidermis a vector from the human keratin K1 gene was constructed. Among its many uses, it is useful in making transgenic animals.

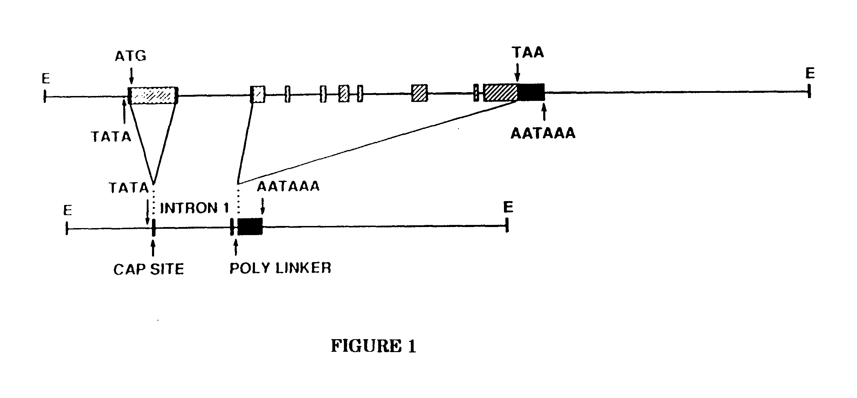

A schematic showing the structure of the human keratin K1 gene is shown in Figure 1. The 12 kb EcoRI fragment containing the entire human keratin K1 gene was originally isolated from lambda clone c55 (Johnson, et al., PNAS, USA, Vol. 82, pp. 1896-1900, (1985)). In constructing the targeting vector, most of the first exon including the ATG was removed, leaving only the 5' non-coding sequences, the first intron and the intron-exon boundaries. In addition, the remainder of the gene up to the termination codon was deleted. A poly linker containing the following unique restriction sites (Bam HI, Xma I, Kpn I, Not I, and Cla I) was engineered into a site 3' of the first intron to allow easy insertion of exogenous DNA. These manipulations were performed through the use of polymerase chain reactions (PCR). The unique EcoRI sites were conserved at the ends of the vector to allow easy amplification in pGEM vectors and excision for purification from plasmid sequences prior to injection into embryos.

The rationale for constructing the vector in this manner was as follows. Since the specific elements responsible for the expression characteristics of the 12 kb human keratin K1 fragment have not been defined, the entire 5' and 3' flanking regions were included in the vector construct. One skilled in the art will readily recognize that as these elements are further defined the flanking sequences can be changed accordingly. In addition, sequences within the 3' non-coding region were retained since these may confirm stability to transcripts of exogenous DNA in epidermal cells. The first intron was retained to potentially enhance expression efficiency (Brinster, et al., PNAS, USA, Vol. 82, pp. 1896-1900 (1988).

### EXAMPLE 2

### HK1 Expression in Epidermal Keratinocytes

To assess the human keratin K1 targeting vector for exclusive expression in epidermal keratinocytes, the β-galactosidase reporter gene was cloned into Bam HI and Cla I restriction sites located in the polylinker region of the expression vector (Figure 1). The β-galactosidase gene has frequently been used as a reporter gene to assess targeting specificity (MacGregor et al., In: Methods in Molecular Biology Vol. 7, pp. 217-235 (1991). This construct was designated pHK1.β-gal. To determine if expression of this construct resulted in the production of a functional protein, and to determine whether the vector retained cell type specificity, this construct was transfected into primary epidermal keratinocytes and primary dermal fibroblasts. At seventy-two hours post transfection cells were stained with a solution containing the substrate 5-bromo-4-chloro-3-indoyl-β-galactosidase (X-gal). β-galactosidase activity, indicated by a blue coloration, was detected in keratinocytes but not fibroblasts. Thus, expression of the HK1.β-gal construct was cell type specific and resulted in the production of a functional protein.

### EXAMPLE 3

### Transgene Mice

The same pHK1.β-gal construct utilized in the *in vitro* studies discussed in Example 2 was used in the production of transgenic mice. This construct was digested with EcoRI (see Figure 1) and subjected to preparative agarose gel electrophoresis to purify the pHK1.β-gal expression construct away from plasmid sequences (pGEM 3) which might interfere with expression. The separated expression construct sequences were purified and recovered using NA 45 DEAE membrane (Schleicher & Schuell). DNA was precipitated and resuspended at 1-3 ng/ul. ICR outbred female mice (Sasco) were given PMS and HCG to stimulate superovulation, mated to FVB males (Taconic) and the resulting early fertilized embryos (most preferably on cell stage) were collected from the oviducts. DNA was micro-injected into the pronuclei and the embryos were surgically transferred to pseudopregnant recipient females (the result of mating ICR females with vasectomized B₆D₂F₁ males (Taconic))

In the initial experiments, 40 mice were born. In order to quickly determine if the pHK1.β-gal transgene was being exclusively expressed in the epidermis of these mice, these animals were sacrificed at birth. A small amount of tissue was removed for extraction of DNA and the remainder of the neonate was rapidly frozen in Tissue-Tek O.C.T. for frozen sections. PCR analysis was performed on the extracted DNA using oligonucleotide primers specific for the intron within the HK1 vector and this demonstrated that 5 of the 40 neonates contained the HK1.β-gal construct.

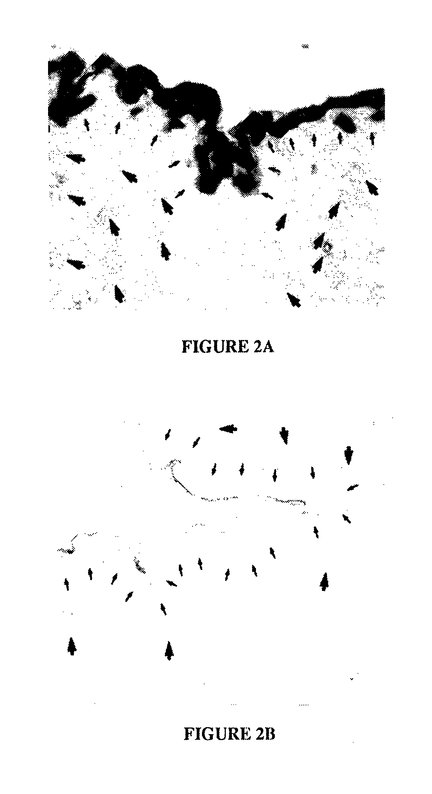

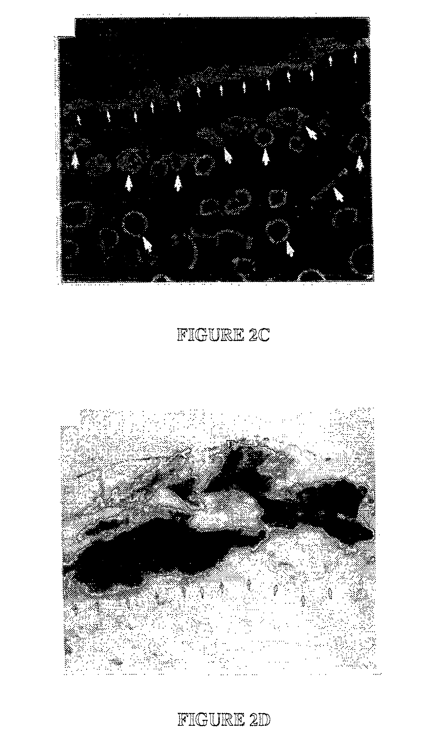

To assess whether expression of the HK1β-gal construct was restricted to the epidermis or expressed in other squamous epithelia, frozen longitudinal sections were cut from several PCR positive and PCR negative embedded neonates and these were stained with X-Gal. Typical results are shown in Figure 2 where PCR positive animal, #30, expressed high levels of β-galactosidase in the epidermis (Figure 2A and a PCR negative sibling, #29, was completely negative (Figure 2B), indicating that endogenous murine β-galactosidase was not expressed at sufficient levels in the epidermis to cause false positives in this assay. Staining of the intestine was observed in both the positive (#30) and negative (#29) neonates. This may represent endogenous enzyme activity or the production of β-galactosidase by bacteria in the intestine. X-gal staining was detected in the basal compartment, although it is not as intense as in the differentiated layers (Figure 2D). Thus, the human keratin K1 expression vector is also expressed in a substantial number of proliferating basal cells.

The most important finding from these initial transgenic experiments in that the vector constructed from the human keratin K1 gene can target the expression of an exogenous coding sequence exclusively to the epidermis of transgenic mice. This specificity of targeting can be readily seen in Figure 2A. This low power exposure of the skin of #30 demonstrates intense staining with X-Gal. In addition, there are numerous hair follicles and sebaceous glands in this section which are marked by arrows and these do not stain with X-Gal. Keratins K5 and K14 are not only expressed in the epidermis, but in all squamous epithelia, including hair follicles and sebaceous glands. The expression pattern for keratin K14 (Figure 2C) is revealed by immunofluorescence with a specific K14 antiserum of an area from a consecutive section that is comparable to that in Figure 2A. Note staining of the epidermis, as well as, hair follicles and sebaceous glands. If the strategy used in construction the human keratin K1 expression vector had altered its targeting specificity in transgenic mice, then X-Gal staining would have been observed in hair follicles, sebaceous glands, other squamous epithelia, and perhaps even other tissue types. However, expression of the HK1.β-gal transgene, like the keratin K1 gene itself is restricted to the epidermis.

### EXAMPLE 4

### Regulation of Keratin K1 Vector by Vitamin D₃

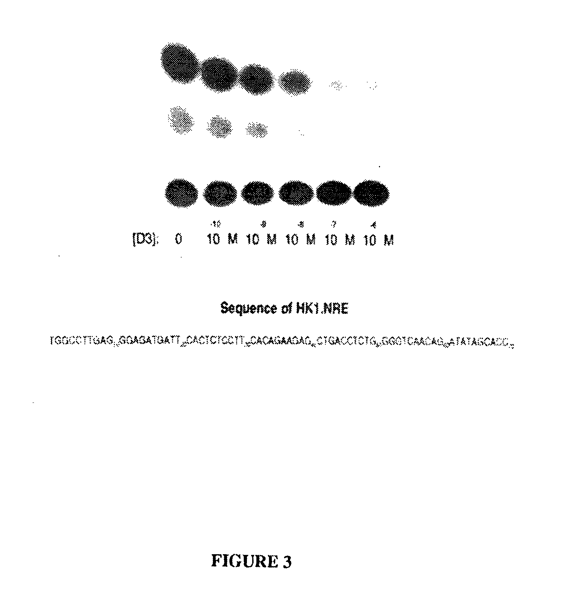

A novel Vitamin D₃ responsive element was used to modulate expression levels in the epidermis. Although all of the regulatory elements of the human keratin K1 gene have not been identified, a novel negative regulatory element from the human keratin K1 gene (HK1.NRE) has been identified and this example demonstrates that it is able to suppress a heterologous promoter in response to Vitamin D₃. The HK1.NRE is 70 nucleotides in length (nucleotides 9134 to 9204 of SEQ. ID. No. 1). PCR technology was used to generate Bam HI and Bgl II sites at opposite ends of this fragment. This facilitates generating multiple copies of this fragment since ligation and digestion with Bam HI and Bgl II will select for oligomers which have ligated head to tail. Four tandem copies of the HK1.NRE were inserted into the Bgl II cloning site of pA10.CAT. In the absence of Vitamin D₃ this construct is highly expressed when transfected into primary mouse epidermal cells (Figure 3). The addition of increasing concentrations of Vitamin D₃ to the culture medium completely suppresses transcription of this heterologous promoter. This observation indicates that the activity of the human keratin K1 expression vector can be modulated in the epidermis. The activity of the human keratin K1 vector is suppressed in the epidermis by topical application of Vitamin D₃, or an analogue, to the skin.

### EXAMPLE 5

### Development of Transgenic Animal Models for Skin Carcinogenesis

The ability to stably introduce genes into the germline of mice has greatly enhanced prospects for generation of animal models of human disease (Leder and Stewart U.S. Patent No. 4,736,866 issued April 12, 1988 and Palmiter and Brinster, Ann. Rev. Genet., Vol. 20, pp. 465-499). When such genes are combined with regulatory sequences that target their expression to specific tissues, it provides a model to not only study diseases in the context of living organisms, but also in specific tissues suspected of being the targets of these genes. Thus, transgenic mice offer the possibility to determine the influence of factors such as blood supply, an intact immune system, humoral and cell-mediated growth controls and physical barriers on disease progression. The epidermis is an attractive tissue for targeted gene expression; not only is it a model for epithelial diseases in general but the accessibility of the epidermis allows easy detection of progressive pathological changes that result from transgene expression as well as the assessment of the potential role played by environmental factors in these processes. In addition, the prospects for utilizing gene therapy to treat cancer are coming closer to reality. Therefore, animal models of human cancers would be useful to assess the therapeutic potential of these approaches. The development of animal models of skin disease is dependent upon the ability to specifically target gene expression to the epidermis. The human keratin K1 targeting vector described in Example 1 is ideally suited for this purpose.

### EXAMPLE 6

### Targeting the v-ras^{Ha} Oncogene to the Epidermis

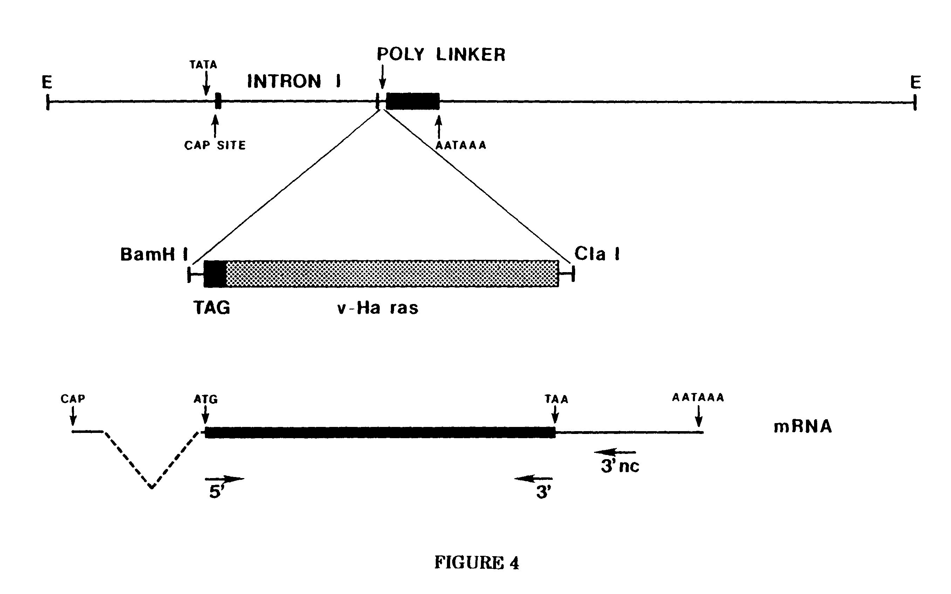

One family of proto-oncogenes, the *ras* family (*ras*^{Ha}, *ras*^{K1}, *ras*^{N}) has been identified in approximately 20% of human tumors by virtue of specific point mutations at codons 12, 13, and 61 which activate their transforming potential. The mechanisms whereby *ras* genes become activated are currently unknown but there is widespread evidence that environmental agents play pivotal roles in the etiology of *ras* mutations. To date few studies have undertaken to study *ras* activation in human skin malignancies. However recent reports have identified *ras*^{Ha} activation in basal and squamous cell carcinomas appearing on sun exposed body sites, interestingly at potential pyrimidine dimer sites possibly derived from skin exposure to UV irradiation. In the mouse skin model of chemical carcinogenesis where the three distinct stages of initiation, promotion and malignant conversion have been defined, *ras*^{Ha} activation has been found in benign squamous papillomas, the end point of initiation and promotion suggesting an early role for *ras*^{Ha} in skin carcinogenesis. Taken collectively, the above experimental evidence suggests the importance of developing an animal model to further study the mechanism of *ras*^{Ha}-induced skin carcinogenesis. Toward this end, the sequence encoding the v-*ras*^{Ha} protein of Harvey Murine Sarcoma Virus (Dhar, et al., Science, Vol. 217, pp. 934-937, (1982) was cloned into the Bam HI and Cla I sites of the human keratin K1 expression vector (Figure 4) To discriminate expression of the v-*ras*^{Ha} transgene from that of the endogenous *ras* gene, a sequence encoding the human keratin K6 epitope SEQ. ID. No 4 was engineered onto the 5' end of the v-*ras*^{Ha} cassette.

HK1 *ras* transgenic mice exhibit the following phenotype: 1) Newborn transgenic mice expressing v-*ras*^{Ha} (HK1 *ras*) exclusively in the epidermis show distinct wrinkled skin at 48 hours and are smaller than litter mates. 2) Juvenile HK1 *ras* transgenic mice exhibit progressive keratinization which peaks at 14 days. 3) The histotype of newborn HK1 *ras* mice reveals massive epidermal hyperplasia with up to 20 fold thickening of the epidermis. 4) By day 14 this progresses to massive hyperkeratosis. Both histotypes are pre-neoplastic, papillomatous, non dysplastic and exhibit few appendages.

The HK1 *ras* transgenic mice develop benign tumors. Typical lesions appear within 10-12 weeks at single sites. The histotype of these tumors reveals a well differentiated squamous papilloma. Papillomas often appear at sites after wounding. Many of these papillomas are prone to regression. This regression phenomenon suggests that *ras*^{Ha} alone is insufficient to maintain even a benign phenotype and requires further events which may involve roles for additional oncogenes/antioncogenes.

### EXAMPLE 7

### Targeting the fos Oncogene to the Epidermis of Transgenic Mice

Recent *in vitro* studies have shown that the v-*fos* gene can convert to malignancy primary keratinocytes or papilloma cell lines which expressed an activated *ras*^{Ha} (Greenhalgh, et al., PNAS, USA, Vol. 87, pp. 643-647, (1990); Greenhalgh and Yuspa, Mol. Carcinogen., Vol. 1, pp. 134-143 (1988). This suggested that *fos* could play a later role in epidermal carcinogenesis and cooperate with the benign phenotype imparted by activated *ras-*^{Ha} expression. Although this alone was sufficient to initiate the establishment of HK1 *fos* transgenic mice with a view to mate with HK1 *ras* mice, two further studies have identified a role for *fos* in normal epidermal differentiation and thus highlights *fos* as an attractive target for perturbation. Using a c-*fos*/β-gal fusion gene Curran and co-workers (Smeyne et al, Neuron, Vol. 8, pp. 13-23 (1992)) have shown significant *fos* expression in the differentiated layers of the epidermis and (Fisher, et al., Development, Vol. III, pp. 253-258, (1991)) have localized c-*fos* expression to a specific subset of granular cells. Thus *fos* may have an important role in the control of the final stages of keratinocyte differentiation. The putative perturbations of this normal role for c-*fos* in such specialized cells by v-*fos* can only be explored in the context of targeted expression in transgenic mice. In addition the c-*fos* proto-oncogene is known to function as a transcriptional regulator in conjunction with the c-*jun*/AP1 gene product and thus, while targeting *ras*^{Ha} represents studies of membrane signalling on neoplasia, targeting *fos* explores the role of transcriptional control on this process.

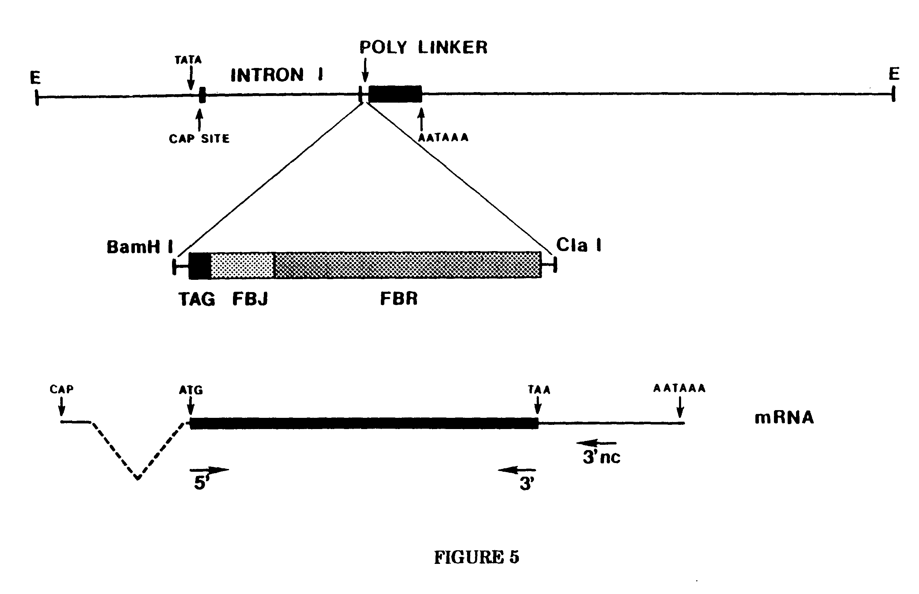

Thus, the *fos* protein coding sequence from the FBJ/FBR chimeric v-*fos* plasmid pFBRJ was inserted into the human keratin K1 targeting vector (Figure 5). To discriminate expression of the v-*fos* transgene from that of the endogenous *fos* gene, a sequence encoding the human keratin K1 epitope (SEQ. ID. No. 5) was engineered onto the 5' end of the v-*fos* cassette.

HK1 *fos* transgenic mice exhibit the following phenotype: 1) A specific ear phenotype typically appears at 3-4 months initially in the wounded (tagged) ear and then becomes bilateral. 2) In several animals expressing severe phenotypes, the wounded ear lesion can grossly resemble a benign keratoacanthoma. 3) Alopecia and hyperkeratosis of the axilla often develop in older animals (approximately 1 year of age).

The histotypes of the HK1 *fos* mice are as follows: 1) The histotype of the initial ear lesions exhibits hyperplasia and hyperkeratosis, a pre-neoplastic pathology with few dysplastic cells and little evidence of further neoplastic progression. 2) At later stages the massive hyperkeratotic histotype resembles a benign keratoacanthoma.

Three HK1 *fos* transgenic mice lines have been established which develop an obvious pre-neoplastic ear phenotype at 3-4 months. The promotion stimulus derived from wounding (i.e. ear tag) appears to accelerate the appearance of this phenotype which eventually becomes bilateral. Also, it appears that friction in the axilla and inguinal area may also promote a pre-neoplastic hyperplastic/hyperkeratotic response after a significant latent period. Collectively these data support a fundamental role for the *fos* gene in normal keratinocyte differentiation and perturbation by v-*fos* results in pre-neoplastic differentiation disorders. In several HK1 *fos* mice severe ear lesions appear to progress to resemble benign keratoacanthomas. Although numbers are low at this time, that this is the resultant tumor type is consistent with a role for *fos* in the latter stages of terminal differentiation, and low numbers and latency suggest a requirement for additional events.

### EXAMPLE 8

### Targeting HPV 18 E6 and E7 Gene Expression to the Epidermis

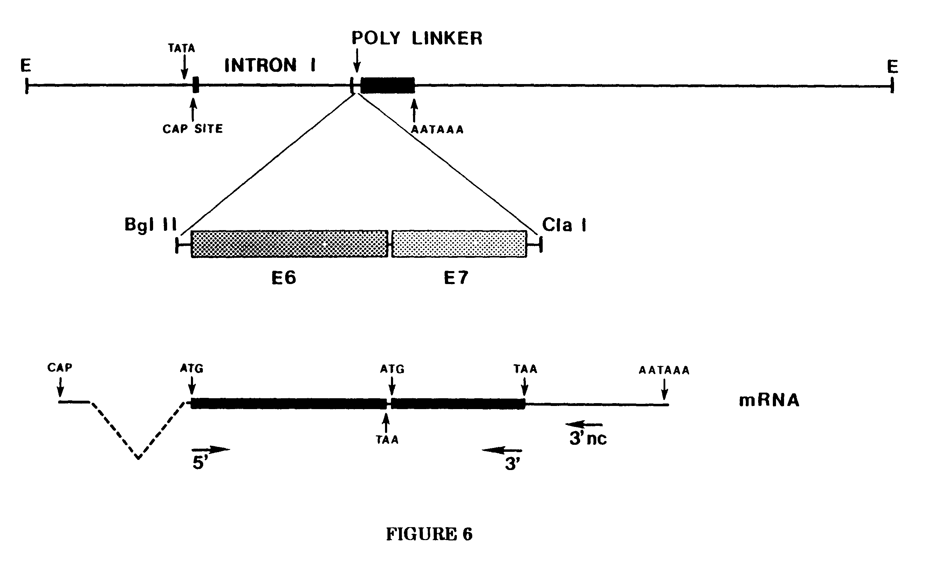

There is widespread evidence from clinical and epidemiological studies which implicate human papilloma viruses (HPV) in the etiology of certain squamous epithelial tumors in humans. HPV's have a specific tropism for squamous epithelial cells and different types of HPVs have specificity for the anatomic site that they infect. Additionally, within a specific subgroup of HPVs, certain types are associated with development of either benign (e.g. HPV6 and 11) or malignant (e.g., HPV-16 and 18) disease and this may center on the properties of the E6 and E7 genes. Through adaptation to the differentiation programs of the epithelia that they infect, HPVs have evolved a clever strategy for the production of infectious progeny. HPVs infect basal epithelial cells but do not undergo lytic replication in this compartment, thus, the germinative pool of cells is not subjected to the cytopathic effects of late viral gene expression. Production of virus only occurs in terminally differentiated cells that have lost proliferative potential and will be desquamated into the environment. This strategy not only provides for the spread of mature viral particles, but ensures their continuous production by replenishment with cells from the basal compartment. Since the life cycle of the virus is so tightly linked to all stages of differentiation of squamous epithelial cells, establishment of successful culture systems has been difficult. To date, these host factors, coupled with regulatory mechanisms present within papilloma virus genomes themselves have also hindered attempts to observe pathological effects of HPV gene expression in squamous epithelia in transgenic mice. These restrictions on utilization of the transgenic mouse model have been overcome with the ability to specifically target HPV gene expression to squamous epithelia using the human keratin K1 targeting vector. In the example provided, the coding sequence for the E6 and E7 genes of HPV 18 were inserted into the human keratin K1 targeting vector at the Bgl II and Cla I sites (Figure 6).

HK1 E6/E7 mice exhibit the following phenotypes and histotypes: 1) One mouse exhibited a subtle skin lesion at 7 months characterized by skin rigidity, thickening and roughness underlying the fur which later progresses to a wart like structure by 10 months. 2) The histotype of this lesion exhibits hyperplasia, hyperkeratosis and the beginnings of verrucous formation. 3) The histotype of a lesion from another mouse at 11 months, is characteristic of a typical wart induced by HPV.

To date three HK1 E6/E7 transgenic mouse lines have been established which develop HPV-like lesions with low frequency and long latent periods. At this time it is unclear whether this limited appearance of phenotypes reflects the subtle nature of the lesion, and its requirement for a long latency period, or the complex nature of HPV biology. However it is noteworthy that our result is consistent with the epidemiology of HPV infections in humans, e.g. although a large percentage of a given female population can test positive for cervical infection by HPV 6, 11, 16, and 18 relatively few progress to develop overt lesions. It may be therefore that the apparent delay and low phenotype frequency exhibited by these mice provides a relevant background to study the consequences of HPV expression during epithelial differentiation. In addition, these mice can be useful in assessing the efficacy of novel antisense pharmaceuticals which have been designed to inhibit expression of the E6 and E7 genes of HPV 18.

### EXAMPLE 9

### Production of Transgenic Mice Expressing TGF-α in the epidermis

Transforming growth factor alpha (TGF-α) is a cytokine with structural and functional characteristics similar to epidermal growth factor (EGF). Both TGF-α and EGF bind to the epidermal growth factor receptor (EGF-R) and stimulate the tyrosine kinase cascade. TGF-α is expressed by both normal and transformed cells and causes proliferation of cultured keratinocytes. *In vivo*, TGF-α induces angiogenesis and is more potent than EGF in accelerating wound healing. In normal human skin, expression of TGF-α occurs in all layers of the epidermis and in certain areas of the appendages. Several cutaneous diseases such as psoriasis, squamous cell carcinoma, and congenital bullous ichthyosiform erythroderma have been associated with altered expression of TGF-α.

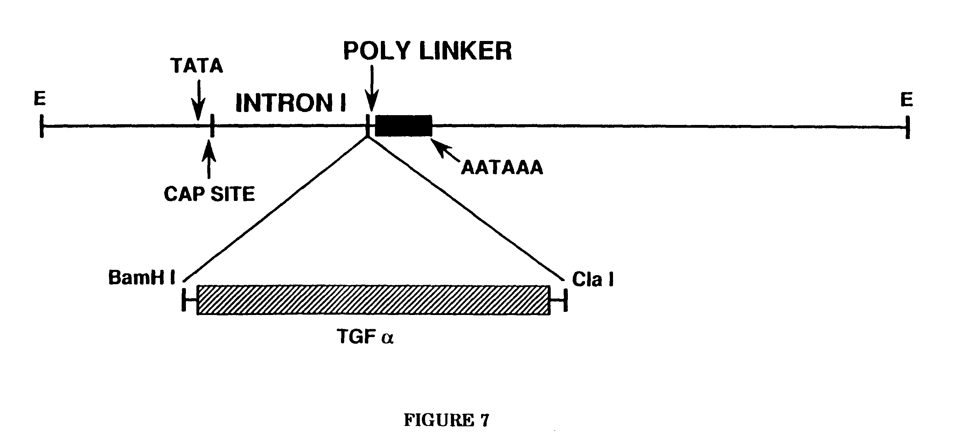

To determine whether altered expression of TGF-α plays a role in the pathogenesis of these diseases, the protein coding sequence of human TGF-α was inserted into the human keratin K1 targeting vector (Figure 7). Injection of the HK1.TGF-α construct into embryos resulted in phenotypic founders that were quite similar to that of *ras*^{Ha}. The histotype was also similar with epidermal hyperplasia, hyperkeratosis and relative alopecia. One founder (2 1/2 months of age) has developed multiple papillomas. Histologically these appear to be squamous papillomas. To date, none of these lesions have converted to a malignant phenotype.

### EXAMPLE 10

### Production of Transgenic Mice Expressing the HIV tat gene in the epidermis.

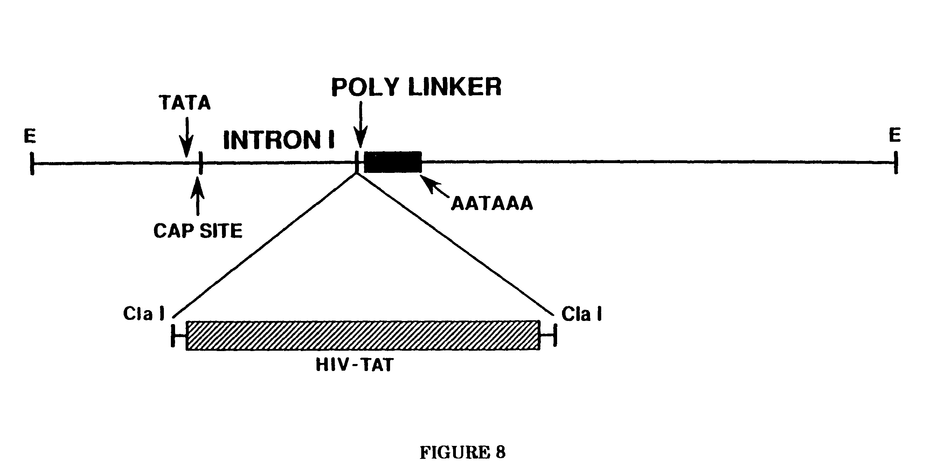

Patients infected with the human immunodeficiency virus (HIV) are at high risk for the development of specific AIDS-associated cutaneous disorders. Often patients manifesting symptoms have skin lesions ranging from hyperproliferative conditions such as psoriasis to Kaposi's sarcoma and metastatic basal cell carcinoma. The precise role of HIV genes, the cells of origin and hence etiology of such skin lesions remains unknown. It may be that specific HIV genes, e.g., the trans-regulatory protein *tat*, play a role directly or indirectly on the homeostatic mechanisms of host cells and tissues. Alternatively, the HIV *tat* gene may interact with or activate other viral genes present from latent or opportunistic infections, e.g., human papilloma virus (HPV). To directly assess the role of keratinocytes in the development of AIDS-associated cutaneous disorders, the HIV *tat* gene is targeted to the epidermis of transgenic mice. Targeting of the *tat* gene and exclusive expression in keratinocytes is achieved by the use of the human keratin K1 vector (Figure 8). The development of strains of mice which develop cutaneous lesions with predictable kinetics as a result of expression of the HIV *tat* gene alone or in combination with other oncogenes serves as a useful model for assessing therapeutic potential of antisense pharmaceuticals designed to inhibit expression of the HIV *tat* gene.

### EXAMPLE 11

### Utilization of the HK1 Vector for Gene Therapy Applications

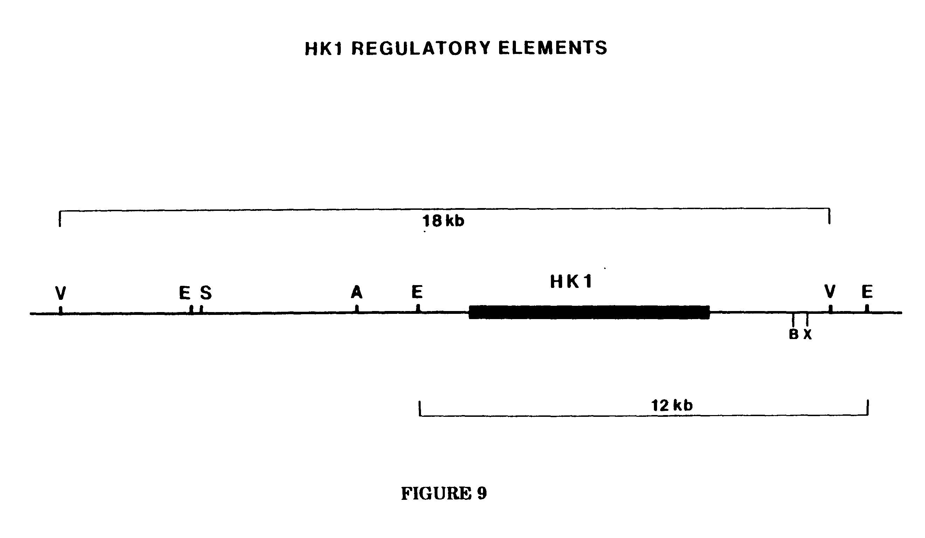

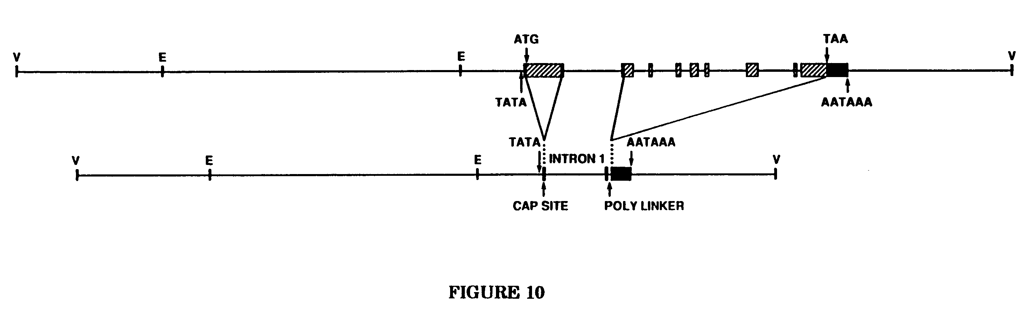

Where exclusive expression in epidermal cells is desirable and for transient expression the HK1 vector is an excellent choice for gene therapy. Unlike the human keratin K1 gene itself, the human keratin K1 vector derived from the 12 kb fragment is expressed in proliferating basal cells in the epidermis. In more recent transgenic experiments, it has been determined that a larger fragment containing the human keratin K1 gene, a 18 kb Eco RV fragment (Shown schematically in Figure 9), is expressed exactly like the endogenous mouse K1 gene, i.e. post mitotically in cells committed to terminal differentiation. These cells are programmed to die and will eventually slough into the environment. Therefore, for human applications where transient expression is desired, it is possible to design a vector that will only be expressed in cells after they commit to terminal differentiation and begin moving upward toward the outer layers of the epidermis. The vector will be expressed approximately 10-14 days prior to being shed into the environment. This can be accomplished by inserting additional 5' flanking sequences from the 18 kb Eco RV fragment onto the end of the original human keratin K1 vector (See Fig. 10).

### EXAMPLE 12

### Detection of Carcinogens and Tumor Promoters

Short-term tests (STTs) for genotoxic chemicals were originally developed as fast, inexpensive assays to assess the potential hazard of chemicals to humans. However, a recent report summarizing the results of a project initiated by the National Toxicology Program to evaluate the ability of STT's to predict rodent carcinogenicity questions the validity of relying solely on STT's. Three of the most potent carcinogens, detected in the rodent assays, produced no genetic toxicity in any of the four STTs evaluated (Tennant et al., Science, Vol. 236, pp. 933-941, (1987). Thus, to receive EPA/FDA approval for new compounds, chemical, agricultural, food and drug companies are currently required to perform two year animal tests costing up to $2 million. The development of new transgenic strains of mice that have been genetically engineered to rapidly detect carcinogens and tumor promoters would substantially reduce the overhead cost of long-term animal studies. The suitability of the transgenic mouse lines claimed in this patent application for rapid detection of carcinogens is initially determined with a known skin carcinogen, DMBA, to determine whether benign lesions appear earlier than in control non-treated litter mates. To determine suitability for detecting tumor promoters, a known promoter, 12-O-tetra-decanoylphorbol-13-acetate (TPA) is applied to *ras*^{Ha}, and *fos* mice. Since benign lesions in *ras*^{Ha} mice and hyperplasia in *fos* mice appeared at sites of wounding (i.e., tagged ears), and wounding can promote tumor formation, these lines are useful for these studies.

All patents and publications mentioned in this specification are indicative of the levels of those skilled in the art to which the invention pertains. All patents and publications which are incorporated herein by reference are incorporated to the same extent as if each individual publication was specifically and individually indicated to be incorporated by reference.

One skilled in the art will readily appreciate that the present invention is well adapted to carry out the objects and obtain the ends and advantages mentioned, as well as those inherent therein. The bioreactors, nucleic acid sequences, transformed epidermal cells, transgenic animals and human keratin K1 vector, along with the methods, procedures, treatments, molecules of specific compounds, are exemplary and are not intended as limitations on the scope of the invention. Changes therein and other uses will occur to those skilled in the art which are encompassed within the spirit of the invention as defined by the scope of the claims.

## Claims

1. A keratin K1 vector for expression of a nucleic acid cassette in the epidermis comprising:
a 5' flanking region of the keratin K1 gene, the 5' flanking sequence including a keratin K1 promoter, a 5' transcribed but untransalated region, and a first intron, all in sequential and positional relationship for expression of the nucleic acid cassette;
a 3' flanking region of the keratin K1 gene containing Vitamin D₃ regulatory sequences, a 3' transcribed but untranslated region and contiguous non-coding DNA containing a transcriptional termination region; and
a polylinker having a plurality of restriction endonuclease sites, the polylinker connecting the 5' flanking region to the 3' flanking region and providing a position for insertion of the nucleic acid cassette, the linker optionally and where necessary additionally comprising the nucleic acid cassette.

2. A vector according to claim 1 wherein the 5' flanking region is approximately 1.2 kb and the 3' flanking region is approximately 3.9 kb.

3. A vector according to claim 1 or 2 wherein the nucleic acid cassette is present and includes a nucleic acid sequence coding for a protein, polypeptide or antisense RNA.

4. A vector according to claim 3 wherein the cassette includes a nucleic acid sequence coding for an oncogene, such as *ras, fos*, *myc*, *erb*, *src*, *sis* or *jun.*

5. A vector according to claim 3 wherein the cassette includes a nucleic acid sequence coding for a transforming gene, the E6 or E7 transforming sequence of human papilloma virus or the TGF-α sequence.

6. A vector according to any preceding claim wherein the restriction endonuclease site is Bam HI, Xma I, Kpn I, Not I, Cla I or Bgl II.

7. A vector according to any preceeding claim further comprising an additional 5' flanking sequence from the 18 kb Eco RV fragment on the end of the vector.

8. A human keratin K1 vector according to claim 1 comprising
a 5' flanking region comprising nucleotides 1 to 1246 of SEQ.ID.No.1;
a 3' flanking region comprising nucleotides 6891 to 10747 of SEQ.ID.No.1; and
a linker comprising nucleotide 2351 to 2376 of SBQ.ID No. 2.

9. An epidermal host cell transformed or transfected with a vector according to any of claims 1 to 8.

10. A host cell according to claim 9 wherein the cassette has a nucleic acid sequence encoding a hormone, a growth factor, an enzyme, a drug, a tumor antigen, a viral antigen, an insect antigen, a bacterial antigen, or a parasitic antigen, for example the sequence encodes proinsulin, insulin, growth hormone, insulin-like growth factor I, insulin-like growth factor II, insulin growth factor binding protein, epidermal growth factor (TGF-α), dermal growth factor (PDGF), an angiogenesis factor, Type IV collagen, laminin, nidogen, Type VII collagen or a protein which induces an immunological response.

11. A bioreactor comprising epidermal cells according to claim 9 or 10.

12. A method of *ex vivo* introduction of a keratin K1 vector into epidermal cells, the method comprising cotransfecting a vector according to any one of claims 3 to 7 with a selectable marker and selecting the transformed cells.

13. A method of making a transgenic non-human animal comprising:
collecting very early fertilized eggs;
inserting a vector according to any one of claims 3 to 7 into the fertilized eggs by micro-injecting the vector into pronuclei; and
transferring the injected eggs into pseudopregnant recipient females.

14. A transgenic non-human animal containing a vector according to any of claims 3 to 7 in its germ and somatic cells, wherein the vector was introduced into the animal or an ancestor of the animal at an embryonic stage and the nucleic acid cassette of the vector is only expressed in the epidermis.

15. A method of studying the origin of, or treatment for, cancer, the method comprising:
making a transgenic non-human animal by injecting an embryo with a human
keratin K1 vector according to any one of claims 3 to 7 and containing an oncogene in the nucleic acid cassette; and
using the resultant animal or the progeny in studies of cancer.

16. A method according to claim 15 wherein the animal contains more than one oncogene.

17. A keratin K1 vector according to any one of claims 3 to 7 and containing a nucleic acid cassette for use for transient introduction of the nucleic acid cassette into a human by contacting an epidermal cell for sufficient time to transduce the cells with the vector.

18. The use of a keratin K1 vector according to claim 3 which includes a nucleic acid cassette having a nucleic acid sequence encoding a growth factor, or an epidermal cell transformed or transduced with such a vector, in the manufacture of a medicament for enhancing healing of a wound or surgical incision or treating skin ulcers.

19. The use according to claim 18 wherein a plurality of vectors are used and wherein the cassette of at least one vector includes the nucleic acid sequence of epidermal growth factor (TGF-α), the cassette of at least one vector includes the nucleic acid sequence of dermal growth factor (PDGF), the cassette of at least one vector includes the nucleic acid sequence for a matrix protein to anchor the epidermis to the dermis or the cassette of at least one vector includes the nucleic acid sequence for an angiogenesis factor.

20. The use according to claim 19 wherein the matrix protein is Type IV collagen, laminin, nidogen, or Type VII collagen and/or the angiogenesis factor is an acid fibroblast growth factor, basic fibroblast growth factor or angiogenin.

21. The use of a keratin K1 vector according to claim 3 which includes a nucleic acid cassette having a nucleic acid sequence encoding for TGF-β, a soluble form of cytokine receptor, or an antisense RNA, or a host epidermal cell transformed or transduced with such a vector, in the manufacture of a medicament for treating psoriasis.

22. The use according to claim 21 wherein the soluble form of cytokine receptor is IL-1, IL-6 or IL-8 and/or the antisense RNA is to a sequence of TGF-α, IL-1, IL-6 or IL-8.

23. The use of keratin K1 vector according to claim 3 which includes a nucleic acid cassette having a nucleic acid sequence encoding for an antisense RNA for the E6 or E7 gene of human papilloma virus or a sequence encoding for the normal p53 protein, or a host epidermal cell transformed or transduced with such a vector, in the manufacture of a medicament for treating skin cancer.

24. The use of a keratin K1 vector according to claim 3 which includes a nucleic acid cassette having a nucleic acid sequence encoding for a protein or polypeptide which induces an immunological response, or a host epidermal cell transformed or transduced with such a vector, in the manufacture of a vaccine.

25. The use according to claim 24 wherein the sequence encodes a viral capsid protein, such as from the human papilloma virus.

26. A pharmaceutical composition comprising:
(a) a vector or epidermal host cell as defined in any of claims 18 to 20, the composition being adapted to enhance healing of a wound or surgical incision or to treat skin ulcers;
(b) a vector or epidermal host cell as defined in claim 21 or 22, the composition being adapted to treat psoriasis:
(c) a vector or epidermal host cell as defined in claim 23 the composition being adopted to treat skin cancer; or
(d) a vector or epidermal host cell as defined in claim 24 the composition being a vaccine.

## Patentansprüche

1. Keratin-K1-Vektor zur Expression einer Nukleinsäurekassette in der Epidermis, umfassend:
eine 5'-flankierende Region des Keratin-K1-Gens, wobei die 5'-flankierende Sequenz einen Keratin-K1-Promotor, eine 5'-transkribierte aber untranslatierte Region und ein erstes Intron einschließt, alle in sequentieller und postionsmäßiger Beziehung für die Expression der Nukleinsäurekassette;
eine 3'-flankierende Region des Keratin-K1-Gens, enthaltend regulatorische Vitamin D₃-Sequenzen, eine 3'-transkribierte aber untranslatierte Region und angrenzende nicht-codierende DNA, enthaltend eine transkriptionelle Terminationsregion; und
einen Polylinker mit einer Mehrzahl von Restriktionsendonuklease-Stellen, wobei der Polylinker die 5'-flankierende Region mit der 3'-flankierenden Region verbindet und eine Position zur Insertion der Nukleinsäurekassette bereitstellt, wobei der Linker wahlfrei und falls notwendig zusätzlich die Nukleinsäurekassette umfasst.

2. Vektor nach Anspruch 1, worin die 5'-flankierende Region ungefähr 1,2 kb groß ist und die 3'-flankierende Region ungefähr 3,9 kb groß ist.

3. Vektor nach Anspruch 1 oder 2, worin die Nukleinsäurekassette vorhanden ist und eine Nukleinsäuresequenz einschließt, welche für ein Protein, Polypeptid oder eine Antisinn-RNA codiert.

4. Vektor nach Anspruch 3, wobei die Kassette eine Nukleinsäuresequenz einschließt, welche für ein Onkogen wie *ras*, *fos*, *myc*, *erb*, *src*, *sis* oder *jun* codiert.

5. Vektor nach Anspruch 3, wobei die Kassette eine Nukleinsäuresequenz einschließt, welche für ein transformierendes Gen, die transformierende E6- oder E7-Sequenz des humanen Papillomvirus oder die TGF-α-Sequenz codiert.

6. Vektor nach mindestens einem vorstehenden Anspruch, wobei es sich bei der Restriktionsendonuklease-Stelle um Bam HI, Xma I, Kpn I, Not I, Cla I oder Bgl II handelt.

7. Vektor nach mindestens einem vorstehenden Anspruch, ferner umfassend eine zusätzliche 5'-flankierende Sequenz aus dem 18 kb großen EcoRV-Fragment am Ende des Vektors.

8. Humaner Keratin-K1-Vektor nach Anspruch 1, umfassend
eine 5'-flankierende Region, umfassend die Nukleotide 1 bis 1246 von SEQ. ID. Nr.1;
eine 3'-flankierende Region, umfassend die Nukleotide 6891 bis 10747 von SEQ. ID. Nr.1; und
einen Linker, umfassend Nukleotid 2351 bis 2376 von SEQ. ID. Nr.2.

9. Epidermale Wirtszelle, transformiert oder transfiziert mit einem Vektor gemäß mindestens einem der Ansprüche 1 bis 8.

10. Wirtszelle nach Anspruch 9, wobei die Kassette eine Nukleinsäuresequenz aufweist, codierend für ein Hormon, einen Wachstumsfaktor, ein Enzym, ein Arzneimittel, ein Tumorantigen, ein virales Antigen, ein Insektenantigen, ein bakterielles Antigen oder ein Parasitenantigen, wobei die Sequenz zum Beispiel für Proinsulin, Insulin, Wachstumshormon, insulinartigen Wachstumsfaktor I, insulinartigen Wachstumsfaktor II, Insulinwachstumsfaktor-bindendes Protein, epidermalen Wachstumsfaktor (TGF-α), dermalen Wachstumsfaktor (PDGF), einen Angiogenese-Faktor, Typ IV-Kollagen, Laminin, Nidogen, Typ VII-Kollagen oder für ein Protein, das eine Immunantwort hervorruft, codiert.

11. Bioreaktor, umfassend epidermale Zellen nach Anspruch 9 oder 10.

12. Verfahren zur *ex vivo*-Einführung eines Keratin-K1-Vektors in epidermale Zellen, wobei das Verfahren das Cotransfizieren eines Vektors nach mindestens einem der Ansprüche 3 bis 7 mit einem selektierbaren Marker und das Selektieren der transformierten Zellen umfasst.

13. Verfahren zur Herstellung eines transgenen, nicht-menschlichen Tieres, umfassend:
Sammeln von sehr frühen, befruchteten Eiern;
Inserieren eines Vektors nach mindestens einem der Ansprüche 3 bis 7 in die befruchteten Eier durch Mikroinjektion des Vektors in Pronuclei; und
Überführen der injizierten Eier in pseudoschwangere Empfängerweibchen.

14. Transgenes, nicht-humanes Tier, enthaltend einen Vektor nach mindestens einem der Ansprüche 3 bis 7 in seinen Keim- und Somazellen, wobei der Vektor bei einem embryonalen Stadium in das Tier oder einen Vorfahren des Tieres eingeführt wurde und die Nukleinsäurekassette des Vektors nur in der Epidermis exprimiert wird.

15. Verfahren zur Untersuchung des Ursprungs oder zur Behandlung von Krebs, wobei das Verfahren umfasst:
Herstellen eines transgenen, nicht-humanen Tieres durch Injizieren eines Embryos mit einem humanen Keratin-K1-Vektor gemäß mindestens einem der Ansprüche 3 bis 7, der ein Onkogen in der Nukleinsäurekassette enthält; und
Verwenden des resultierenden Tieres oder der Nachkommen in Krebsuntersuchungen.

16. Verfahren gemäß Anspruch 15, wobei das Tier mehr als ein Onkogen enthält.

17. Keratin-K1-Vektor nach mindestens einem der Ansprüche 3 bis 7 und enthaltend eine Nukleinsäurekassette zur Verwendung für die transiente Einführung der Nukleinsäurekassette in einen Menschen durch Kontaktieren einer epidermalen Zelle während einer ausreichenden Zeit, um die Zellen mit dem Vektor zu transduzieren.

18. Verwendung eines Keratin-K1-Vektors gemäß Anspruch 3, welche eine Nukleinsäurekassette, die eine für einen Wachstumsfaktor codierende Nukleinsäuresequenz aufweist, oder eine epidermale Zelle, transformiert oder transduziert mit einem derartigen Vektor, bei der Herstellung eines Medikaments zur Förderung der Heilung einer Wunde oder eines chirurgischen Schnitts oder zur Behandlung von Hautgeschwüren einschließt.

19. Verwendung nach Anspruch 18, wobei eine Mehrzahl von Vektoren verwendet wird und wobei die Kassette von mindestens einem Vektor die Nukleinsäuresequenz von epidermalem Wachstumsfaktor (TGF-α) einschließt, die Kassette von mindestens einem Vektor die Nukleinsäuresequenz von dermalen Wachstumsfaktor (PDGF) einschließt, die Kassette von mindestens einem Vektor die Nukleinsäuresequenz für ein Matrixprotein zur Verankerung der Epidermis an der Dermis einschließt, oder die Kassette von mindestens einem Vektor die Nukleinsäuresequenz für einen Angiogenese-Faktor einschließt.

20. Verwendung gemäß Anspruch 19, wobei das Matrixprotein Typ IV-Kollagen, Laminin, Nidogen oder Typ VII-Kollagen ist und/oder der Angiogenese-Faktor ein saurer Fibroblasten-Wachstumsfaktor, basischer Fibroblasten-Wachstumsfaktor oder Angiogenin ist.

21. Verwendung eines Keratin-K1-Vektors gemäß Anspruch 3, welche eine Nukleinsäurekassette, aufweisend eine für TGF-β, eine lösliche Form von Cytokinrezeptor oder eine Antisinn-RNA codierende Nukleinsäuresequenz, oder eine epidermale Wirtszelle, transformiert oder transduziert mit einem derartigen Vektor, bei der Herstellung eines Medikaments zur Behandlung von Psoriasis einschließt.

22. Verwendung gemäß Anspruch 21, wobei die lösliche Form von Cytokinrezeptor IL-1, IL-6 oder IL-8 ist und/oder die Antisinn-RNA gegen eine Sequenz von TGF-α, IL-1, IL-6 oder IL-8 gerichtet ist.

23. Verwendung eines Keratin-K1-Vektors gemäß Anspruch 3, welche eine Nukleinsäurekassette, aufweisend eine für eine Antisinn-RNA für das E6- oder E7-Gen von humanem Papillomvirus codierende Nukleinsäuresequenz oder eine für das normale p53-Protein codierende Sequenz, oder eine epidermale Wirtszelle, transformiert oder transduziert mit einem derartigen Vektor, bei der Herstellung eines Medikaments zur Behandlung von Hautkrebs einschließt.

24. Verwendung eines Keratin-K1-Vektors gemäß Anspruch 3, welche eine Nukleinsäurekassette, aufweisend eine für ein Protein oder Polypeptid, das eine Immunantwort hervorruft, codierende Nukleinsäuresequenz, oder eine epidermale Wirtszelle, transformiert oder transduziert mit einem derartigen Vektor, bei der Herstellung eines Impfstoffs einschließt.

25. Verwendung gemäß Anspruch 24, wobei die Sequenz für ein virales Capsidprotein codiert, wie aus dem humanen Papillomvirus.

26. Pharmazeutische Zusammensetzung, umfassend:
(a) Vektor oder epidermale Wirtszelle, wie definiert in mindestens einem der Ansprüche 18 bis 20, wobei die Zusammensetzung zur Förderung der Heilung einer Wunde oder eines chirurgischen Schnitts oder zur Behandlung von Hautgeschwüren ausgelegt ist;
(b) Vektor oder epidermale Wirtszelle, wie definiert in Anspruch 21 oder 22, wobei die Zusammensetzung zur Behandlung von Psoriasis ausgelegt ist;
(c) Vektor oder epidermale Wirtszelle, wie definiert in Anspruch 23, wobei die Zusammensetzung zur Behandlung von Hautkrebs ausgelegt ist; oder
(d) Vektor oder epidermale Wirtszelle, wie definiert in Anspruch 24, wobei es sich bei der Zusammensetzung um einen Impfstoff handelt.

## Revendications

1. Vecteur de kératine K1 pour l'expression dune cassette d'acide nucléique dans l'épiderme, comprenant :
une région flanquante 5' du gène de kératine K1, la séquence flanquante 5' incluant un promoteur de kératine K1, une région 5' transcrite mais non traduite, et un premier intron, tous en relation séquentielle et de position pour l'expression de la cassette d'acide nucléique ;
une région flanquante 3' du gène de kératine K1 contenant des séquences régulatrices de vitamine D₃, une région 3' transcrite mais non traduite et un ADN non codant contigu contenant une région de terminaison de la transcription ; et
un lieur multisite ayant une pluralité de sites d'endonucléase de restriction, le lieur multisite reliant la région flanquante 5' à la région flanquante 3' et fournissant une position pour l'insertion de la cassette d'acide nucléique, le lieur, éventuellement et lorsque cela est nécessaire, comprenant en plus la cassette d'acide nucléique.

2. Vecteur selon la revendication 1, dans lequel la région flanquante 5' est d'environ 1,2 kb et la région flanquante 3' est d'environ 3,9 kb.

3. Vecteur selon la revendication 1 ou 2, dans lequel la cassette d'acide nucléique est présente et inclut une séquence d'acide nucléique codant une protéine, un polypeptide ou un ARN antisens.

4. Vecteur selon la revendication 3, dans lequel la cassette inclut une séquence d'acide nucléique codant un oncogène, tel que *ras, fos*, *myc, erb*, *src, sis* ou *jun.*

5. Vecteur selon la revendication 3, dans lequel la cassette inclut une séquence d'acide nucléique codant un gène transformant, la séquence transformante E6 ou E7 du virus de papillome humain ou la séquence de TGF-α.

6. Vecteur selon l'une quelconque des revendications précédentes, dans lequel le site d'endonucléase de restriction est Bam HI, Xma I, Kpn I, Not I, Cla I ou Bgl II.

7. Vecteur selon l'une quelconque des revendications précédentes, comprenant en outre une séquence flanquante 5' supplémentaire du fragment Eco RV de 18 kb à l'extrémité du vecteur.

8. Vecteur de kératine K1 humaine selon la revendication 1, comprenant :
une région flanquante 5' comprenant les nucléotides 1 à 1246 de SEQ. ID. No.1 ;
une région flanquante 3' comprenant les nucléotides 6891 à 10747 de SEQ.ID. No.1 ; et
un lieur comprenant les nucléotides 2351 à 2376 de SEQ. ID. No.2.

9. Cellule hôte épidermique transformée ou transfectée par un vecteur selon l'une quelconque des revendications 1 à 8.

10. Cellule hôte selon la revendication 9, dans laquelle la cassette a une séquence d'acide nucléique codant une hormone, un facteur de croissance, une enzyme, un médicament, un antigène tumoral, un antigène viral, un antigène d'insecte, un antigène bactérien, ou un antigène parasitaire, par exemple, la séquence code une pro-insuline, une insuline, une hormone de croissance, le facteur de croissance I analogue à l'insuline, le facteur de croissance II analogue à l'insuline, une protéine de liaison au facteur de croissance insulinique, le facteur de croissance épidermique (TGF-α), un facteur de croissance dermique (PDGF), un facteur d'angiogénèse, le collagène de type IV, la laminine, le nidogène, le collagène de type VII ou une protéine qui induit une réponse immunologique.

11. Bioréacteur comprenant des cellules épidermiques selon la revendication 9 ou 10.

12. Méthode d'introduction *ex vivo* d'un vecteur de kératine K1 dans des cellules épidermiques, la méthode comprenant les étapes consistant à co-transfecter un vecteur selon l'une quelconque des revendications 3 à 7 avec un marqueur sélectionnable et à sélectionner les cellules transformées.

13. Méthode d'obtention d'un animal non-humain transgénique comprenant les étapes consistant :
à récolter des oeufs fertilisés à un stade très précoce ;
à insérer un vecteur selon l'une quelconque des revendications 3 à 7 dans les oeufs fertilisés par micro-injection du vecteur dans les pronoyaux ; et
à transférer les oeufs injectés dans des femelles receveuses pseudo-gravides.

14. Animal non-humain transgénique contenant un vecteur selon l'une quelconque des revendications 3 à 7 dans ses cellules germinales et somatiques, le vecteur ayant été introduit dans l'animal, ou un ancêtre de l'animal à un stade embryonnaire, et la cassette d'acide nucléique du vecteur n'étant exprimée que dans l'épiderme.

15. Méthode d'étude de 1'origine, ou du traitement, du cancer, la méthode comprenant les étapes consistant :
à produire un animal non-humain transgénique par injection dans un embryon d'un vecteur de kératine K1 humain selon l'une quelconque des revendications 3 à 7 et contenant un oncogène dans la cassette d'acide nucléique ; et
à utiliser l'animal obtenu ou sa descendance dans des études sur le cancer.

16. Méthode selon la revendication 15, dans laquelle l'animal contient plus d'un oncogène.

17. Vecteur de kératine K1 selon l'une quelconque des revendications 3 à 7, et contenant une cassette d'acide nucléique, destiné à être utilisé pour l'introduction transitoire de la cassette d'acide nucléique dans un humain en mettant en contact une cellule épidermique pendant une durée suffisante pour transduire les cellules avec le vecteur.

18. Utilisation d'un vecteur de kératine K1 selon la revendication 3, qui inclut une cassette d'acide nucléique ayant une séquence d'acide nucléique codant un facteur de croissance, ou une cellule épidermique transformée ou transduite par un tel vecteur, dans la fabrication d'un médicament pour l'amélioration de la cicatrisation d'une plaie ou d'une incision chirurgicale ou pour le traitement d'ulcères cutanés.

19. Utilisation selon la revendication 18, dans laquelle une pluralité de vecteurs est utilisée et dans laquelle la cassette d'au moins un vecteur inclut la séquence d'acide nucléique du facteur de croissance épidermique (TGF-α), la cassette d'au moins un vecteur inclut la séquence d'acide nucléique du facteur de croissance dermique (PDGF), la cassette d'au moins un vecteur inclut la séquence d'acide nucléique pour une protéine matrice permettant d'ancrer l'épiderme au derme ou la cassette d'au moins un vecteur inclut la séquence d'acide nucléique pour un facteur d'angiogénèse.

20. Utilisation selon la revendication 19, dans laquelle la protéine matrice est du collagène de type IV, de la laminine, du nidogène, ou du collagène de type VII et/ou le facteur d'angiogénèse est un facteur de croissance des fibroblastes acide, un facteur de croissance des fibroblastes basique ou l'angiogénine.

21. Utilisation d'un vecteur de kératine K1 selon la revendication 3, qui inclut une cassette d'acide nucléique ayant une séquence d'acide nucléique codant TGF-β, une forme soluble d'un récepteur de cytokine, ou un ARN antisens, ou une cellule épidermique hôte transformée ou transduite avec un tel vecteur, dans la fabrication d'un médicament pour le traitement du psoriasis.

22. Utilisation selon la revendication 21, dans laquelle la forme soluble du récepteur de cytokine est IL-1, IL-6 ou IL-8 et/ou l'ARN antisens est contre une séquence de TGF-α, IL-2, IL-6 ou IL-8.

23. Utilisation de vecteur de kératine K1 selon la revendication 3, qui inclut une cassette d'acide nucléique ayant une séquence d'acide nucléique codant un ARN antisens pour le gène E6 ou E7 du virus de papillome humain ou une séquence codant la protéine p53 normale, ou une cellule épidermique hôte transformée ou transduite par un tel vecteur, dans la fabrication d'un médicament pour le traitement du cancer de la peau.

24. Utilisation d'un vecteur de kératine K1 selon la revendication 3, qui inclut une cassette d'acide nucléique ayant une séquence d'acide nucléique codant une protéine ou un polypeptide qui induit une réponse immunologique, ou une cellule épidermique hôte transformée ou transduite par un tel vecteur, dans la fabrication d'un vaccin.

25. Utilisation selon la revendication 24, dans laquelle la séquence code une protéine de capside virale, telle que provenant du virus de papillome humain.

26. Composition pharmaceutique comprenant :
(a) un vecteur ou une cellule hôte épidermique tel que défini dans l'une quelconque des revendications 18 à 20, la composition convenant à l'amélioration de la cicatrisation d'une plaie ou d'une incision chirurgicale, ou au traitement d'ulcères cutanés ;
(b) un vecteur ou une cellule hôte épidermique tel que défini dans la revendication 21 ou 22, la composition convenant au traitement du psoriasis ;
(c) un vecteur ou une cellule hôte épidermique tel que défini dans la revendication 23, la composition convenant au traitement du cancer de la peau ; ou
(d) un vecteur ou une cellule hôte épidermique tel que défini dans la revendication 24, la composition étant un vaccin.


### SEQUENCE LISTING

(1) GENERAL INFORMATION:
   (i) APPLICANT:
      Roop, Dennis R.
      Rothnagel, Joseph A.
      Greenhalgh, David A.
      Yuspa, Stuart H.
   (ii) TITLE OF INVENTION: DEVELOPMENT OF A VECTOR TO TARGET GENE EXPRESSION TO THE EPIDERMIS OF TRANSGENIC ANIMALS
   (iii) NUMBER OF SEQUENCES: 5
   (iv) CORRESPONDENCE ADDRESS:
      (A) ADDRESSEE: Fulbright & Jaworski
      (B) STREET: 1301 McKinney, Suite 5100
      (C) CITY: Houston
      (D) STATE: Texas
      (E) COUNTRY: U.S.A.
      (F) ZIP: 77010-3095
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US
      (B) FILING DATE:
      (C) CLASSIFICATION:
   (viii) ATTORNEY/AGENT INFORMATION:
      (A) NAME: Paul, Thomas D.
      (B) REGISTRATION NUMBER: 32,714
      (C) REFERENCE/DOCKET NUMBER: D-5478
   (ix) TELECOMMUNICATION INFORMATION:
      (A) TELEPHONE: 713/651-5325
      (B) TELEFAX: 713/651-5246
      (C) TELEX: 762829
(2) INFORMATION FOR SEQ ID NO:1:
   (i) SEQUENCE CHARACTERISTICS:
      (A) LENGTH: 10747 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:1:
   GAATTCGGCT GCTGTGCTGT CGTACAACAT GCTGTTTAGG ATCTTGCACA TGATAGCTAG   60
   GTATTCTTGC TTCAAATCGC AGGCACCCCA CTTACCAACT GTGTAGACTT GATCACGTTA  120
   TTCAACCCCT GTGTCTCTGC TTCCTCATTT TACAAATGGG GAGAAAAATA GCATCTATCT  180
   CAAAGTTGTG AAAATTAAGC AAGTTAATAC ATATGTGCTA CGTAGAACAG TGCCTGGTAC  240
   ATGGTCAGTT TTTGATACAT GTTAGGTATT ATCATTATTA TCACCTCCAG AAACAATTTA  300
   AACTTCTCAT ATAAGGCTCT CCAGACACCT CTCATTGTCT TCCCTTCCAA ATCTGCATTT  360
   ATCTCTCTCT CTTTGCAGTC CAGTGTGAGG CTTGAATCAC CTATCAAGCC TCACCTCCAC  420
   CCCTGTGCTT TACAAAATGT CCTAGAGCTT CTATTTACTC GTCTCACTGC TCTGTGGGCT  480
   TTTTCACTCA AGGGCGTTTG CATGCTATCC ATTGCTACCT GTTTTCTGTT GCTGGTGTCT  540
   GTCTCCTGCT CTATCTTTGA AGAAAAGAAA CAAGAAAAGG AATAACTGAG AAACAGAGAA  600
   AAAAAATGTC TCTCCCTTCT GGTTCTTCCA GACCACCCAC TCATCCATCT TGTTCAATGA  660
   CAGCTTCTCT TCCTTTAATT AATCACTGTG GTATATTTAT AAAGCTTATA TTTATGAAAG  720
   ACCTTTTAAT TTTTTAGTTA TTAAAGCCCT TTCTCTTTGT CAGGTTGTAA CTGAGTGAGC  780
   TCTGGAGTTT GGAAAGAAGA TCTTAGAAAT GGGCCAGAGA GCTCCTTCTG AGATCCAAGC  840
   ACGGAGAATT GCACCTGCTG TGCATGGTAA GAGAGTGTGC TTGGTAGCTC ACAAGGGCAA  900
   GGTGAGAATA GAAACTTTCA TGCCTTTTTG ATGGGGGTTA TGAAATCCTA CCAAGAAACA 960
   CCAGGTATCA GATGTGGGGT CCTGTTTTCC CAAAGCCACA AATGCTTGAA GGAAGATCTT 1020
   GTGTGATAAA ATAATTACCA CATGAACCAA TCTTGCATGC ACAGCAATTT TGAGAGCCCA 1080
   TCCTGGGAGC TAGGTGTGTA GTGTTTATCG TATTGTTGAG GCTCGTAAAA ATCTTGTATG 1140
   GCTGCAGGCA AGCCAAACCC TTGACAGGCA CTGCATCTCC GCTGACTCTA GAAGACCAAG 1200
   CCCAATTTCT TCCCTGTATA TAAGGGGAAG TCTCTATGCT TGGGGTAGAG GAGTGTTTAG 1260
   CTCCTTCCCT TACTCTACCT TGCTCCTACT TTTCTCTAAG TCAACATGAG TCGACAGTTT 1320
   AGTTCCAGGT CTGGGTACCG AAGTGGAGGG GGCTTCAGCT CTGGCTCTGC TGGGATCATC 1380
   AACTACCAGC GCAGGACCAC CAGCAGCTCC ACACGCCGCA GTGGAGGAGG TGGTGGGAGA 1440
   TTTTCAAGCT GTGGTGGTGG TGGTGGTAGC TTTGGTGCTG GTGGTGGATT TGGAAGTCGG 1500
   AGTCTTGTTA ACCTTGGTGG CAGTAAAAGC ATCTCCATAA GTGTGGCTAG AGGAGGTGGA 1560
   CGTGGTAGTG GCTTTGGTGG TGGTTATGGT GGTGGTGGCT TTGGTGGTGG TGGCTTTGGT 1620
   GGTGGTGGCT TTGGTGGAGG TGGCATTGGG GGTGGTGGCT TTGGTGGTTT TGGCAGTGGT 1680
   GGTGGTGGTT TTGGTGGAGG TGGCTTTGGG GGTGGTGGAT ATGGGGGTGG TTATGGTCCT 1740
   GTCTGCCCTC CTGGTGGCAT ACAAGAAGTC ACTATCAACC AGAGCCTTCT TCAGCCCCTC 1800
   AATGTGGAGA TTGACCCTGA GATCCAAAAG GTGAAGTCTC GAGAAAGGGA GCAAATCAAG 1860
   TCACTCAACA ACCAATTTGC CTCCTTCATT GACAAGGTGA GTTTCTCTCT CATTGCACTG 1920
   GTAGGGCTGC CGCTGGTCCA CTTGGGATTG GTGCAGTCAA AACACATGTA GGTTTGAACC 1980
   TCAAGTTTCC ATGTTTACAT GATTAAAAGG ATGTTTTGTG GAATGGTCTC CTAGGAGATA 2040
   TGTTAGATGT ATGCTTGTGA ATGGTGTTAA TGACTCTCTC TTTGACAAAG GGTTCGTGGT 2100
   CGACCTAAAG GTGGGTCAGT GTGACATTAA CATTTAAGTG CTTTTTATTC AGCTCTTGAG 2160
   CGGAATTGGG ACTCATATCT GTTGAATGAA GATAATAGAA ATGGGGCTAA CTGAACTTTC 2220
   CAGGGTGCAA GTGAGAACCC TGGAAAGGTC TTCCTAACCA TAGAAAGGGA GTTGAGTGTG 2280
   AACATAGTAT AGAGTGTTAT TGTAGCAGAA AACATGTGGT CAGTCAGTGC CAAACATCTT 2340
   TTGCTGTCAG AGGGGAGCTC TGCCTTCTAA TAATTTTACA TTGGTACTGG ATGAGGCTAG 2400
   AGTTTTTTTA TACTAATATC TCCAAAAATC AGCTCTAAAA AACTCAGATA AACCATTTTT 2460
   TTAATTTTTT GCTTAATCAT TAATAGTGCC AATCCAAGGT TATCCACAAC AAATTTCAAA 2520
   TCCAATTTTG AATTTTCCTG ATATACTTTT GAAATGTGTG TGTGTCCTGG GGATGCAAAC 2580
   CAGTTTTTAT GGTAATATAC CTAACAAAAT TTTGGAAGGC AAATCTCTTA AATACCATGC 2640
   ACCTATTTCA AAACATAATT GCAATAATTC TGTATGCGCT TTGCTATTGG TATTTGTTTA 2700
   GTTACTCCCT TCCAAGCCCT CTCTGAATTA ACAAGTTGGG TTTTATTATG CAGATGATAT 2760
   TAACTTGATC ATCTTCTTCC TATTTCTCTG TCATGGTCAG AAGATAGGAA TTGAGGTTCT 2820
   TTTCCAAATG AGGCACAGTT CTCCATGGCT ATGAGACTCC ATTTATGCAT CAGGAGTAAA 2880
   GGGGTCTTGT GTTTTTAGGT GAGGTTCCTG GAGCAGCAGA ACCAGGTACT GCAAACAAAA 2940
   TGGGAGCTGC TGCAGCAGGT AGATACCTCC ACTAGAACCC ATAATTTAGA GCCCTACTTT 3000
   GAGTCATTCA TCAACAATCT CCGAAGGAGA GTGGACCAAC TGAAGAGTGA TCAATCTCGG 3060
   TTGGATTCGG AACTGAAGAA CATGCAGGAC ATGGTGGAGG ATTACCGGAA CAAGTAAGGG 3120
   ACCCTGTCTG GGCAGTTCTT AACTTTTGCT GTAAAAGAGT TCCAGAAAGT AATAAGCTAA 3180
   GATCATGAAG CAGCATGTAG CTATGTCTTT TCTTAGGTTA GAGGCACATC AGTTTGACAT 3240
   TTTCAGAAAT CTTCATTTTC TCAGGAGATG GAAATAGTCT AGTGGTTTTA TTGCTCAGTA 3300
   GAAAGTAGTG GCCAATATGT CCTAGGTTCA TAATAGAAAG GCAGTGATAG GCAATGCCAC 3360
   CTTTAGTTTA GAATGCTGGA CTTCAGGTCT TACCACCTCT GAATCTCCTA ATTGTTTCTG 3420
   CTTTCCTGCA GGTATGAGGA TGAAATCAAC AAGCGGACAA ATGCAGAGAA TGAATTTGTG 3480
   ACCATCAAGA AGGTAAGCAA ATTCTGTAGG ACGGAACTCA CATTTGAAAT AAATAAGGGA 3540
   AGAGGGTCTC CAATTACTAA GCAGAAAGCA GCCATGATAT GGAGAGCCAG GTAGTAGACC 3600
   TGGGGAGTAT ATGGAGTGGG GCTATATTTT TCACATCATC ATGGACCTGG ACTGATCCAG 3660
   GCACTTGGCT TCTCCATATT TCCCAGCACC TTACATAGTA AGTGGAGTGG CAGATTCTCA 3720
   GCAAGCCAGG CACACTCCCT TGATGGTGCT ATCCGGGGGT GGGACAGTTA GGGAACTGTG 3780
   ATTTACCTGG GGCAAAAAGG AGTGGAGTAG ACCCAAAGCT CCTTTTTTTG CTTGGAGAAT 3840
   CCCCTCACAG GTAATGAGAG GGACCTGCCC TGGAGAGAAC GTGCCTTCAT GATGTCCCTT 3900
   GTTCCTCTAG GATGTGGATG GTGCTTATAT GACCAAGGTG GACCTTCAGG CCAAACTTGA 3960
   CAACTTGCAG CAGGAAATTG ATTTCCTTAC AGCACTCTAC CAAGCAGTAA GTCTTCCAGT 4020
   TTCAACCAAG TTTATCTAAA TGGAGAGTTT TTAAGCCGGA ACCCACAACG ATTCAGAAGA 4080
   ATAGATATTT ATCTTTTATT TCCTGACTGC TTTCTCTGTC TAAGTTGTTT TTTGTTTTAG 4140
   TGCTGTAAGA GTCACTAACC TATTATGTCT TGCAGGAGTT GTCTCAGATG CAGACTCAAA 4200
   TCAGTGAAAC TAATGTCATC CTCTCTATGG ACAACAACCG CAGTCTCGAC CTGGACAGCA 4260
   TCATTGCTGA GGTCAAGGCC CAGTACGAGG ATATAGCCCA GAAGAGCAAA GCTGAGGCCG 4320
   AGTCCTTGTA CCAGAGCAAG GTGAGTGGGC TGAAACCGTA GCCAGTTTCC CTGAAATGGC 4380
   TTGTCTTGCT ATCCTGTGTT ATCTCATGTA TGTGTGCCTG TGCCATGCTG AGTTCTGCCT 4440
   ACATTTAACA AACGCTATCT ACCATCTTTA GTATGAAGAG CTGCAGATCA CTGCTGGCAG 4500
   ACATGGGGAT AGTGTGAGAA ATTCAAAGAT AGAAATTTCT GAGCTGAATC GTGTGATCCA 4560
   GAGACTTAGA TCTGAAATCG ACAATGTCAA GAAGCAGGTA TGTGCTTTCT CCTTCTACCA 4620
   CTCAGCTGTA TGGAATGGGG GTAACCCTCA GGTAAAGGGC GAGTGCTTTC CTAGTTTTGA 4680
   ATCTTGCAAT TCAGCCCAAG GCTACATTAT TAGCCCTGGT TCCTTTTCTG ACTATGCTAG 4740
   TTTCCAGAAT GCAGCCATCA TGCTGGGTTC TCTTTAGGGA AATCTGTGAG AATGGCCTAG 4800
   TAGAGAAAGA TGGGATGGTC AATGTGAGTG ATCTAGCCTA TGACCCAAAG TGGACTTAAG 4860
   AGTTGGGGAG TGAGAGGAAG GGCAGCCAGG AGGTTTTAGA GTAGGTGTTT AGAAGAATGT 4920
   CAAGTCTGTA AGGGTTGTAG GAGCCTTGAC TCAGGGCCAA GAGAGGCTGT TGAGTTATCC 4980
   CTAAGGTCTT TTAAGGAAGT CAACATGGTG ATGTGTTATC TGGAGGTGGG TGTGAGATGA 5040
   CTTAAGGCCA AGTGGTTCTG TTGGACTCAT TATTGGCCTC ACTGGAGTGG GGAGACCAAT 5100
   TGGGATGAGG AGGCCTAGTG GGGAATGCAT ATTATGAGAG GGTGTCATAT CTTTTTCAGA 5160
   TCTCCAACTT GCAGCAGTCC ATCAGTGATG CAGAGCAGCG TGGCGAGAAT GCCCTCAAGG 5220
   ATGCCAAGAA CAAGCTGAAT GACCTGGAGG ATGCCCTGCA GCAGGCCAAG GAAGACCTGG 5280
   CCCGCCTGCT GCGTGACTAC CAGGAGCTGA TGAACACCAA GCTGGCCCTG GATCTGGAGA 5340
   TTGCCACCTA CAGGACCCTC CTGGAGGGAG AAGAAAGCAG GTGAGGAAGG GACGCTGGGA 5400
   GTCGAACCTC TTCTCATGGT CTTCCTTCCT TGCAAGCTGA TTGTTGTTGA AGATGCAGCC 5460
   ATCTGATTGC AGCTTGTGCT GGGTATGGGG AAATGAAAAG TACACGGAGC AGGAGGAAGG 5520
   ACCTAGTTTT ACTTTGGGAG CTGGAGTCCC AAGCTGTTTA TTTTTTTCTT CTAGGGCTGT 5580
   AACATATCTA GAAAGAGCTT TGAGGTGGAG CAAATTATTC TTTATCTGGG CTGCCTCAGA 5640
   TGGCAGCTGG CCTAAAGTCG GCATCTTTAG AGGGGGCCTT CATTGGCTGC AAGGCTCGTC 5700
   TCGTTTATAT GGGAATTTCT CCGTGTTTGT ACTCTTGCTG AGAAAAAATG ACAGGTCTGG 5760
   GAGGCCAGAG GGGATTGGAT TAAGTTTCAG ATTAAGTGCA TTGGAGAAGA CCCAGATGGG 5820
   GAAAGTCTTC AAGGTGGTGG AGCGGGGAAT GGGGAAGCGG TTTGGGAAGC TGGAGTGTCC 5880
   TGAGGAATTT TCTTATTTTC TCCTACAGGA TGTCTGGAGA ATGTGCCCCG AACGTGAGTG 5940
   TGTGTAAGTA CAAGTCGATT TCTCAGGGGC ATGTGCAGGC TTTGTTGGGC TGGAAACGGA 6000
   GTTGAGGTTG AAAATAACTG AGCTTCCTCT TGCAGCTGTG AGCACAAGCC ACACCACCAT 6060
   CAGTGGAGGT GGCAGCCGAG GAGGTGGCGG CGGTGGCTAC GGCTCTGGAG GTAGCAGCTA 6120
   TGGCTCCGGA GGTGGTAGCT ATGGTTCTGG AGGTGGCGGC GGCGGCGGCC GTGGCAGCTA 6180
   TGGCTCCGGA GGTGGCAGCT ATGGCTCTGG AGGTGGCGGC GGCGGCCATG GCAGCTACGG 6240
   CTCCGGAAGC AGCAGTGGGG GCTACAGAGG TGGCTCTGGA GGCGGCGGCG GCGGCAGCTC 6300
   TGGCGGCCGG GGCTCTGGCG GCGGGAGCTC TGGAGGCTCC ATAGGAGGCC GGGGATCCAG 6360
   CTCTGGGGGT GTCAAGTCCT CTGGTGGCAG TTCCAGCGTG AAGTTTGTTT CTACCACTTA 6420
   TTCCGGAGTA ACCAGATAAA GAGATGCCCT CTGTTTCATT AGCTCTAGTT CTCCCCCAGC 6480
   ATCACTAACA AATATGCTTG GCAAGACCGA GGTCGATTTG TCCCAGCCTT ACCGGAGAAA 6540
   AGAGCTATGG TTAGTTACAC TAGCTCATCC TATTCCCCCA GCTCTTTCTT TTCTGCTGTT 6600
   TCCCAATGAA GTTTTCAGAT CAGTGGCAAT CTCAGTCCCC TGGCTATGAC CCTGCTTTGT 6660
   TCTTTCCCTG AGAAACAGTT CAGCAGTGAC CACCACCCAC ATGACATTTC AAAGCACCTC 6720
   CTTAAGCCAG CCAGAGTAGG ACCAGTTAGA CCCAGGGTGT GGACAGCTCC TTAGCATCTT 6780
   ATCTCTGTGC TGTTTTGGTT TTGTACATAA GGTGTAAGCA AGTTGTTTTT CTTTTGTGGA 6840
   GAGGTCTTAA ACTCCCCATT TCCTTGTTTT GCTGCAATAA ACTGCATTTG AAATTCTCCA 6900
   TGTCTCGATC GCCCTTGTTT ACGGCACTGT CTAACCTGGA TGGGTGTTTT GTGAGGTAAA 6960
   AGAAGACACT AGAGCCACAT GGCATATGGG AAAGTCATGC ACACAAACAT GAGAAAAATG 7020
   CAGAGGCCAA CCAGGCAACA TTTCACCAGA CTGGAATCAC AGAGAGAGCA AGCACTTTCC 7080
   CAGATGGTGG GGATGTCATG GAGAAATGGA GAGACCGGGT GACAGGTTTT GTTCATTTGA 7140
   GAAGGCTTTC TTGAAAAGGG CAGTGAGCAA GCAGGTTGGG AGGAAGAGGT GTGGCATTGA 7200
   GAAGAAGGGA AAGTATTGCA TGAAAAAGTA ATTCTTCACG TGGAACAGCC AGTAAGGAGG 7260
   GGCATGAGTA ATATAGGGTC AGCAGTTACT GGAGCCAGAA TACAGACTTT GGCCTGGGGA 7320
   GTTCAAGAAC TAAGAGTGGT AATAGAGAGT TGGATATTCC ATTTCCCTTC TCTTTTTGTG 7380
   CCACCACCCA AAGCTCTGCA TAATCTAAGA AGTTCCCTTG TTGACACATA GCTCATACTT 7440
   GTGAAGTTGT ACAACAGGAT AGCATAGTGG CCAGAAGCAT GGACAGTTGA ACTCAGATAT 7500
   GCTTGGGTTT GAATCTTACC ATCACCATTT ACTAGTTCTG TAATACAGTG CAAGTTACAG 7560
   ACATCTCTGC ACCTCAGTTT TAGTATGTCT AAATTGGGGA TGATAATGCC TTCCTTGTGG 7620
   GGATAGTGTG AGGATTGAAT AAGATGAATA CACATGGCTG AGCACACAGC AAGCACTAAA 7680
   TAAGTGCCAG TTTTAATGAT AACGGTGATG ATGATGATGA TGATGATGAT GACGTAACAT 7740
   TGCTTGTGGG ACTCCATACA GCTCAGTAGA TGCTTGCTCA AAGAAGCAAG TTACCAAAAT 7800
   TTTTGTAATG GTTCTATGAA CGTGAAAAAA GCAGTCAACT TCTCTGAGGA TCAATTTCCT 7860
   TAGTTTCCAA TTAGGAAAAG TCTTCTTAGC TCCAGAGTCC CACAGGGCTA ATGGAATAAG 7920
   GAGAGGATAG ATCACACATG TATTATGCAA ACACAACTCA GGTGAGCTCT ATTCTTCCTT  7980
   CTCAGTTATC CCTTCTGTAG GGACCCCAGT GTCCCCTGCT GTCTTTCTGT GTCCTGACCG  8040
   GGAAACACAG TGTGCCTTGT CTACTCCATC ACTTGGCCAG CTGCATGCTT TCCTTTGCAG  8100
   GCTTGAAGCA AAGCTGGGTC TCGGACATTC TCAGGCACTG ACAAAGCTGT TTAGTTGTTG  8160
   CTGGGAAACA CTGGGAAATA GCCCTTTTGT TAAACACACA GAAACTAGCC TTCGCCCTGA  8220
   GCCAAATTCC TTAAACTCGT CTATGAAATT CCATAACCTG ACTCCTTAAC TGCAGACATA  8280
   CCCAGCTAGA ACATCCCTCA TGTCCCTGTC CACCGTGAGA ATGCTGCACT TCACTCTGAA  8340
   CCTTTAGTCC TCCTTTTAAA TACTGCACAC TGATCACCCT GGTGTTTAGT GCTTTGTTTT  8400
   TTGGAATCCC ACCTGGCTCC ATTTTGGGAT GGTTCCGGGC ACTTCCCTAT GGAAATTCCC  8460
   CTGCTGTCAC TGTCAGAGTG AGTCCAGCAG TGGGTTTAGC TGGATGAAAC ACCACCATGT  8520
   CCATTTCCAT TCAGACTAAT GTCAGAATTT GAAAGGCACT ATGGTAGAGT AGAAAGAACA  8580
   AGGAACTGTA CTATTTAAAG GGCAGGCAAA GAAAAGGCAT CTATAGCTTA TAAGATGTGT  8640
   GGATCTTTGG ATGTGACTTG GCCATCCTGA GCCTAAGTTG TCTTGTAGGA GAAATGGGAA  8700
   TGAGAATATT TTCCTCTAGA CATCAAGAGG AAAAGAAATA TAACGTGAAA ACCTTTGTGA  8760
   ATTGTGAATG TGTTATACAG AGTAGCTAAA AGAATTAAAA AGGGAGTGAC AAAAAAGTAA  8820
   AAGGCAGCTG GCTGCTCAGG GCCTCCATGG AGGGAAGTAC CTTGATATGG TCACTGTGGC  8880
   TCAGTGACAG CTCTGCAGGG ACAGGAAATT GATTTGTTAG TGCACCCAAA GTTGAATCTG  8940
   CTCCTGAGTA CTGATTTATG GGAACCAAAC ACACAAGAGA TGAAGGATGT GTCAACCAGA  9000
   ATGTCCAGCA TTAGCTTGTG GGGAAACACA TACTTCCAGT GACTGAAATA CCATCCTGTT  9060
   ATCAAGAGAT CTGGGAAACT AAAGTACTGA CAAGAGCTGG CTTGATCTGT GGATTTAGAA  9120
   CAATGAGAGT TAGGTGGCCT TGAGGGAGAT GATTCACTCT CCTTCACAGA AGAGCTGACC  9180
   TCTGGGGTCA ACAGATATAG CACCTCTTTC CCAGGGACGC TACTGAATGA ACAGTGATGT  9240
   GTTCTTATAC TCTGGCCCAG ATTTTCTACA TACTTTCTTA GGTTACAACT TTATTTAGTC  9300
   ACATTTCAGT ACTGGGGATA CTCCTGTTTA TCTTCTTTGG ACTCGAGTTT TTATGGGAAG  9360
   GTCATGAAAC AGAGAAAAAT ACAATTTGCA GGGAAACTTA CCAAGGCTTG TAAGGTTACA  9420
   AGGATTAAAT GAAAACCCTG TGTAAGTCAG TATATAGTGA AGAAGTAAAT TGAGTTAGAC  9480
   CAAACGCCAA AATGCATCCG CATTAGAAAG ACGATAAAGG AAGACTCTGG ATTCAGTTCT  9540
   GTTCAAAAAA CATTTTCTGC ACAAATACTA TGTATGAGGA ACTGGGCGTT GGGGAGATGA  9600
   TGATGAGTGA GACATGGTTC TTGCTTTCAG AGAGCCTAGA GACCTGGGTG GTAGCAATGG  9660
   TAGAGATACA TCCAAGACAC AGAAATAGAT ATACAGGAAC ACAGATGATT GAAAGTGATG  9720
   CTTGGCAGGG CTTTAAAGAA TGAATCAGAG TTTTTCAGGC AGACGAGGAT CTTCAAGGCA  9780
   GAGGGAATCA TATAGATAAG GACATAGAAG AGTGAAATTT CATGAAGTAG TTAAGCATCT  9840
   GAAGAAGCAT GGAATTAGTG ACAAGAAATG ATGCGGAAAA GATATCCAGA TCCAATCAAG  9900
   AAGGGCCTTG TTGGCATTCT ATGGAGTCTG GACTTTGGCT TCTGGGTCAC AAGTTCTCAG  9960
   ATGGGGTTTT CATATCTATT ATTAGACCTA CTATGTACTG GTCCAGTGGA AGGGAAAGGG 10020
   GTTGTCTTAC TGCTAGTGGA GTAGGAATTG GGTATGGACC ACAGCTTGTC TTGTTTCCAA 10080
   GTATTCCCTA AGAAATCTGG TCTGCTGATG GGAGATCTAT TTATGGAAAT GTCTTTTTCC 10140
   CTCAGGAATT TTATGTCGGA AACAGCTGTC ATAGGTGAGG AGGAACTGGT AAAAGTACTT 10200
   AATAGGAGAG TGTCATGGTC AGATTGGTGT TTTGGAAAAG TCAGCCAGGG CAGATTGGAG 10260
   AGGTCCATAT TGGAGGCAGG AAGACTTAAG AGACTATTGC AAAGGTGAAG ACAAAAGACG 10320
   ATAGGGACTT GCACTTTAAT TCCAGCCCTT AGAAGTAGTA GAAGGTCAGA AATGAGAATA 10380
   TGCATTACAG AGATAGTTAG TTGCTATATC ATTAGGACTT GGTGATAGAT TGGATGAGGA 10440
   TGCGGTTGGG TGAGGCAAAG AGGAGAGTCC ACATTCCTGG TCTGGGTAGT AACAAAGAAT 10500
   CTAGCAAGAG GGCTTGTGGG GAAAGATGCT GAGTTACGTA GCAAGTGCAT CTGCTTTATC 10560
   CTTGTAATGA ATGGGGCTAA AGGTGTAAAC CAAAGAGTCA TCAGCATTTG GAGGGTAGAA 10620
   TAAATCATCA GATAACTCAG GAAGAAGGAG CAGAAGAATT ACTGATACTC CCTGGAAGGA 10680
   AAACCGGAAG TAAATGGGAG AAACTTGCTC AAGTGGACAA AGTTTAACAG ACATGAAGCA 10740
   TGAATTC                                                          10747
(2) INFORMATION FOR SEQ ID NO:2:
   (i) SEQUENCE CHARACTERISTICS:
      (A) LENGTH: 6693 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:2:
   GAATTCGGCT GCTGTGCTGT CGTACAACAT GCTGTTTAGG ATCTTGCACA TGATAGCTAG   60
   GTATTCTTGC TTCAAATCGC AGGCACCCCA CTTACCAACT GTGTAGACTT GATCACGTTA  120
   TTCAACCCCT GTGTCTCTGC TTCCTCATTT TACAAATGGG GAGAAAAATA GCATCTATCT  180
   CAAAGTTGTG AAAATTAAGC AAGTTAATAC ATATGTGCTA CGTAGAACAG TGCCTGGTAC  240
   ATGGTCAGTT TTTGATACAT GTTAGGTATT ATCATTATTA TCACCTCCAG AAACAATTTA  300
   AACTTCTCAT ATAAGGCTCT CCAGACACCT CTCATTGTCT TCCCTTCCAA ATCTGCATTT  360
   ATCTCTCTCT CTTTGCAGTC CAGTGTGAGG CTTGAATCAC CTATCAAGCC TCACCTCCAC  420
   CCCTGTGCTT TACAAAATGT CCTAGAGCTT CTATTTACTC GTCTCACTGC TCTGTGGGCT  480
   TTTTCACTCA AGGGCGTTTG CATGCTATCC ATTGCTACCT GTTTTCTGTT GCTGGTGTCT  540
   GTCTCCTGCT CTATCTTTGA AGAAAAGAAA CAAGAAAAGG AATAACTGAG AAACAGAGAA  600
   AAAAAATGTC TCTCCCTTCT GGTTCTTCCA GACCACCCAC TCATCCATCT TGTTCAATGA  660
   CAGCTTCTCT TCCTTTAATT AATCACTGTG GTATATTTAT AAAGCTTATA TTTATGAAAG 720
   ACCTTTTAAT TTTTTAGTTA TTAAAGCCCT TTCTCTTTGT CAGGTTGTAA CTGAGTGAGC 780
   TCTGGAGTTT GGAAAGAAGA TCTTAGAAAT GGGCCAGAGA GCTCCTTCTG AGATCCAAGC 840
   ACGGAGAATT GCACCTGCTG TGCATGGTAA GAGAGTGTGC TTGGTAGCTC ACAAGGGCAA 900
   GGTGAGAATA GAAACTTTCA TGCCTTTTTG ATGGGGGTTA TGAAATCCTA CCAAGAAACA 960
   CCAGGTATCA GATGTGGGGT CCTGTTTTCC CAAAGCCACA AATGCTTGAA GGAAGATCTT 1020
   GTGTGATAAA ATAATTACCA CATGAACCAA TCTTGCATGC ACAGCAATTT TGAGAGCCCA 1080
   TCCTGGGAGC TAGGTGTGTA GTGTTTATCG TATTGTTGAG GCTCGTAAAA ATCTTGTATG 1140
   GCTGCAGGCA AGCCAAACCC TTGACAGGCA CTGCATCTCC GCTGACTCTA GAAGACCAAG 1200
   CCCAATTTCT TCCCTGTATA TAAGGGGAAG TCTCTATGCT TGGGGTAGAG GAGTGTTTAG 1260
   CTCCTTCCCT TACTCTACCT TGCTCCTACT TTTCTCTAAG TCAACATCGA ATTTGCCTCC 1320
   TTCATTGACA AGGTGAGTTT CTCTCTCATT GCACTGGTAG GGCTGCCGCT GGTCCACTTG 1380
   GGATTGGTGC AGTCAAAACA CATGTAGGTT TGAACCTCAA GTTTCCATGT TTACATGATT 1440
   AAAAGGATGT TTTGTGGAAT GGTCTCCTAG GAGATATGTT AGATGTATGC TTGTGAATGG 1500
   TGTTAATGAC TCTCTCTTTG ACAAAGGGTT CGTGGTCGAC CTAAAGGTGG GTCAGTGTGA 1560
   CATTAACATT TAAGTGCTTT TTATTCAGCT CTTGAGCGGA ATTGGGACTC ATATCTGTTG 1620
   AATGAAGATA ATAGAAATGG GGCTAACTGA ACTTTCCAGG GTGCAAGTGA GAACCCTGGA 1680
   AAGGTCTTCC TAACCATAGA AAGGGAGTTG AGTGTGAACA TAGTATAGAG TGTTATTGTA 1740
   GCAGAAAACA TGTGGTCAGT CAGTGCCAAA CATCTTTTGC TGTCAGAGGG GAGCTCTGCC 1800
   TTCTAATAAT TTTACATTGG TACTGGATGA GGCTAGAGTT TTTTTATACT AATATCTCCA 1860
   AAAATCAGCT CTAAAAAACT CAGATAAACC ATTTTTTTAA TTTTTTGCTT AATCATTAAT 1920
   AGTGCCAATC CAAGGTTATC CACAACAAAT TTCAAATCCA ATTTTGAATT TTCCTGATAT 1980
   ACTTTTGAAA TGTGTGTGTG TCCTGGGGAT GCAAACCAGT TTTTATGGTA ATATACCTAA 2040
   CAAAATTTTG GAAGGCAAAT CTCTTAAATA CCATGCACCT ATTTCAAAAC ATAATTGCAA 2100
   TAATTCTGTA TGCGCTTTGC TATTGGTATT TGTTTAGTTA CTCCCTTCCA AGCCCTCTCT 2160
   GAATTAACAA GTTGGGTTTT ATTATGCAGA TGATATTAAC TTGATCATCT TCTTCCTATT 2220
   TCTCTGTCAT GGTCAGAAGA TAGGAATTGA GGTTCTTTTC CAAATGAGGC ACAGTTCTCC 2280
   ATGGCTATGA GACTCCATTT ATGCATCAGG AGTAAAGGGG TCTTGTGTTT TTAGGTGAGG 2340
   TTCCTGGAGC AGGATCCCGG GTACCGCGGC CGCATCGATT CGATAAGAGA TGCCCTCTGT 2400
   TTCATTAGCT CTAGTTCTCC CCCAGCATCA CTAACAAATA TGCTTGGCAA GACCGAGGTC 2460
   GATTTGTCCC AGCCTTACCG GAGAAAAGAG CTATGGTTAG TTACACTAGC TCATCCTATT 2520
   CCCCCAGCTC TTTCTTTTCT GCTGTTTCCC AATGAAGTTT TCAGATCAGT GGCAATCTCA 2580
   GTCCCCTGGC TATGACCCTG CTTTGTTCTT TCCCTGAGAA ACAGTTCAGC AGTGACCACC 2640
   ACCCACATGA CATTTCAAAG CACCTCCTTA AGCCAGCCAG AGTAGGACCA GTTAGACCCA 2700
   GGGTGTGGAC AGCTCCTTAG CATCTTATCT CTGTGCTGTT TTGGTTTTGT ACATAAGGTG 2760
   TAAGCAAGTT GTTTTTCTTT TGTGGAGAGG TCTTAAACTC CCCATTTCCT TGTTTTGCTG 2820
   CAATAAACTG CATTTGAAAT TCTCCATGTC TCGATCGCCC TTGTTTACGG CACTGTCTAA 2880
   CCTGGATGGG TGTTTTGTGA GGTAAAAGAA GACACTAGAG CCACATGGCA TATGGGAAAG 2940
   TCATGCACAC AAACATGAGA AAAATGCAGA GGCCAACCAG GCAACATTTC ACCAGACTGG 3000
   AATCACAGAG AGAGCAAGCA CTTTCCCAGA TGGTGGGGAT GTCATGGAGA AATGGAGAGA 3060
   CCGGGTGACA GGTTTTGTTC ATTTGAGAAG GCTTTCTTGA AAAGGGCAGT GAGCAAGCAG 3120
   GTTGGGAGGA AGAGGTGTGG CATTGAGAAG AAGGGAAAGT ATTGCATGAA AAAGTAATTC 3180
   TTCACGTGGA ACAGCCAGTA AGGAGGGGCA TGAGTAATAT AGGGTCAGCA GTTACTGGAG 3240
   CCAGAATACA GACTTTGGCC TGGGGAGTTC AAGAACTAAG AGTGGTAATA GAGAGTTGGA 3300
   TATTCCATTT CCCTTCTCTT TTTGTGCCAC CACCCAAAGC TCTGCATAAT CTAAGAAGTT 3360
   CCCTTGTTGA CACATAGCTC ATACTTGTGA AGTTGTACAA CAGGATAGCA TAGTGGCCAG 3420
   AAGCATGGAC AGTTGAACTC AGATATGCTT GGGTTTGAAT CTTACCATCA CCATTTACTA 3480
   GTTCTGTAAT ACAGTGCAAG TTACAGACAT CTCTGCACCT CAGTTTTAGT ATGTCTAAAT 3540
   TGGGGATGAT AATGCCTTCC TTGTGGGGAT AGTGTGAGGA TTGAATAAGA TGAATACACA 3600
   TGGCTGAGCA CACAGCAAGC ACTAAATAAG TGCCAGTTTT AATGATAACG GTGATGATGA 3660
   TGATGATGAT GATGATGACG TAACATTGCT TGTGGGACTC CATACAGCTC AGTAGATGCT 3720
   TGCTCAAAGA AGCAAGTTAC CAAAATTTTT GTAATGGTTC TATGAACGTG AAAAAAGCAG 3780
   TCAACTTCTC TGAGGATCAA TTTCCTTAGT TTCCAATTAG GAAAAGTCTT CTTAGCTCCA 3840
   GAGTCCCACA GGGCTAATGG AATAAGGAGA GGATAGATCA CACATGTATT ATGCAAACAC 3900
   AACTCAGGTG AGCTCTATTC TTCCTTCTCA GTTATCCCTT CTGTAGGGAC CCCAGTGTCC 3960
   CCTGCTGTCT TTCTGTGTCC TGACCGGGAA ACACAGTGTG CCTTGTCTAC TCCATCACTT 4020
   GGCCAGCTGC ATGCTTTCCT TTGCAGGCTT GAAGCAAAGC TGGGTCTCGG ACATTCTCAG 4080
   GCACTGACAA AGCTGTTTAG TTGTTGCTGG GAAACACTGG GAAATAGCCC TTTTGTTAAA 4140
   CACACAGAAA CTAGCCTTCG CCCTGAGCCA AATTCCTTAA ACTCGTCTAT GAAATTCCAT 4200
   AACCTGACTC CTTAACTGCA GACATACCCA GCTAGAACAT CCCTCATGTC CCTGTCCACC 4260
   GTGAGAATGC TGCACTTCAC TCTGAACCTT TAGTCCTCCT TTTAAATACT GCACACTGAT 4320
   CACCCTGGTG TTTAGTGCTT TGTTTTTTGG AATCCCACCT GGCTCCATTT TGGGATGGTT 4380
   CCGGGCACTT CCCTATGGAA ATTCCCCTGC TGTCACTGTC AGAGTGAGTC CAGCAGTGGG 4440
   TTTAGCTGGA TGAAACACCA CCATGTCCAT TTCCATTCAG ACTAATGTCA GAATTTGAAA 4500
   GGCACTATGG TAGAGTAGAA AGAACAAGGA ACTGTACTAT TTAAAGGGCA GGCAAAGAAA 4560
   AGGCATCTAT AGCTTATAAG ATGTGTGGAT CTTTGGATGT GACTTGGCCA TCCTGAGCCT 4620
   AAGTTGTCTT GTAGGAGAAA TGGGAATGAG AATATTTTCC TCTAGACATC AAGAGGAAAA 4680
   GAAATATAAC GTGAAAACCT TTGTGAATTG TGAATGTGTT ATACAGAGTA GCTAAAAGAA 4740
   TTAAAAAGGG AGTGACAAAA AAGTAAAAGG CAGCTGGCTG CTCAGGGCCT CCATGGAGGG 4800
   AAGTACCTTG ATATGGTCAC TGTGGCTCAG TGACAGCTCT GCAGGGACAG GAAATTGATT 4860
   TGTTAGTGCA CCCAAAGTTG AATCTGCTCC TGAGTACTGA TTTATGGGAA CCAAACACAC 4920
   AAGAGATGAA GGATGTGTCA ACCAGAATGT CCAGCATTAG CTTGTGGGGA AACACATACT 4980
   TCCAGTGACT GAAATACCAT CCTGTTATCA AGAGATCTGG GAAACTAAAG TACTGACAAG 5040
   AGCTGGCTTG ATCTGTGGAT TTAGAACAAT GAGAGTTAGG TGGCCTTGAG GGAGATGATT 5100
   CACTCTCCTT CACAGAAGAG CTGACCTCTG GGGTCAACAG ATATAGCACC TCTTTCCCAG 5160
   GGACGCTACT GAATGAACAG TGATGTGTTC TTATACTCTG GCCCAGATTT TCTACATACT 5220
   TTCTTAGGTT ACAACTTTAT TTAGTCACAT TTCAGTACTG GGATACTCCC TGTTTATCTT 5280
   CTTTGGACTC GAGTTTTTAT GGGAAGGTCA TGAAACAGAG AAAAATACAA TTTGCAGGGA 5340
   AACTTACCAA GGCTTGTAAG GTTACAAGGA TTAAATGAAA ACCCTGTGTA AGTCAGTATA 5400
   TAGTGAAGAA GTAAATTGAG TTAGACCAAA CGCCAAAATG CATCCGCATT AGAAAGACGA 5460
   TAAAGGAAGA CTCTGGATTC AGTTCTGTTC AAAAAACATT TTCTGCACAA ATACTATGTA 5520
   TGAGGAACTG GGCGTTGGGG AGATGATGAT GAGTGAGACA TGGTTCTTGC TTTCAGAGAG 5580
   CCTAGAGACC TGGGTGGTAG CAATGGTAGA GATACATCCA AGACACAGAA ATAGATATAC 5640
   AGGAACACAG ATGATTGAAA GTGATGCTTG GCAGGGCTTT AAAGAATGAA TCAGAGTTTT 5700
   TCAGGCAGAC GAGGATCTTC AAGGCAGAGG GAATCATATA GATAAGGACA TAGAAGAGTG 5760
   AAATTTCATG AAGTAGTTAA GCATCTGAAG AAGCATGGAA TTAGTGACAA GAAATGATGC 5820
   GGAAAAGATA TCCAGATCCA ATCAAGAAGG GCCTTGTTGG CATTCTATGG AGTCTGGACT 5880
   TTGGCTTCTG GGTCACAAGT TCTCAGATGG GGTTTTCATA TCTATTATTA GACCTACTAT 5940
   GTACTGGTCC AGTGGAAGGG AAAGGGGTTG TCTTACTGCT AGTGGAGTAG GAATTGGGTA 6000
   TGGACCACAG CTTGTCTTGT TTCCAAGTAT TCCCTAAGAA ATCTGGTCTG CTGATGGGAG 6060
   ATCTATTTAT GGAAATGTCT TTTTCCCTCA GGAATTTTAT GTCGGAAACA GCTGTCATAG 6120
   GTGAGGAGGA ACTGGTAAAA GTACTTAATA GGAGAGTGTC ATGGTCAGAT TGGTGTTTTG 6180
   GAAAAGTCAG CCAGGGCAGA TTGGAGAGGT CCATATTGGA GGCAGGAAGA CTTAAGAGAC 6240
   TATTGCAAAG GTGAAGACAA AAGACGATAG GGACTTGCAC TTTAATTCCA GCCCTTAGAA 6300
   GTAGTAGAAG GTCAGAAATG AGAATATGCA TTACAGAGAT AGTTAGTTGC TATATCATTA 6360
   GGACTTGGTG ATAGATTGGA TGAGGATGCG GTTGGGTGAG GCAAAGAGGA GAGTCCACAT 6420
   TCCTGGTCTG GGTAGTAACA AAGAATCTAG CAAGAGGGCT TGTGGGGAAA GATGCTGAGT 6480
   TACGTAGCAA GTGCATCTGC TTTATCCTTG TAATGAATGG GGCTAAAGGT GTAAACCAAA 6540
   GAGTCATCAG CATTTGGAGG GTAGAATAAA TCATCAGATA ACTCAGGAAG AAGGAGCAGA 6600
   AGAATTACTG ATACTCCCTG GAAGGAAAAC CGGAAGTAAA TGGGAGAAAC TTGCTCAAGT 6660
   GGACAAAGTT TAACAGACAT GAAGCATGAA TTC 6693
(2) INFORMATION FOR SEQ ID NO:3:
   (i) SEQUENCE CHARACTERISTICS:
      (A) LENGTH: 24979 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:3:
   GAATTCCAAG CTTTCTGCTG TAAGGAGGGA CCTCAGGGAG CCAAGGTCAG CCTGCAGCCT    60
   TTCTGTGCTC CTTTGCCTCG CCTGACAGGT ATGAGGATGA AATCAACAAG AGGACTGGCA   120
   GCGAGAATGA CTTTGTCGTC CTGAAGAAGG TGAGGGAAAG GGGAGTCCTG AGGGTGGCTG   180
   TGGACCCAGG AGGCTGAGGG GAGTGAGGAA TCCCTATGGA TGCTCTGTGA CAATGGCAGG   240
   GTGGCCTCTA CGGCCGGCTT GCTGTGTATG ATGCCTGAAT GCGGGGCCCT TACATTGGAA   300
   CTGACACTGA TAATGACTCT TCAGGAAGCC TTGAGTTCGT ATCTCTCTGG GGTCTGAAAG   360
   TGAAATGAAG TGAAATGACA GCTTTTGAGT GTCAGTTACC TGTAGCCTTG GGACCTAAGG   420
   AAGGACCTGG GGTGTTGGTT GTGACTGACT GGGATGTGGA GGTTGGTGTC ACATCTCCTT   480
   CTGGCCAGGA AAGCCAGGAC TTGTGGGTCC TTATTCGAGT GCGGTGATGA ATTTTTTAAG   540
   TAAGGAAATA AACCTAGAGT GGCTCTGGTC CTGAGCCAGC CAGTGAGCTG TGGCAGGCAA   600
   TGCCTGGGCA ATAAAGTCAA ACTGTTCTGC CTGCTATTCA GGATGTGGAT GCTGCTTATG   660
   TGAGCAAAGT GGACCTGGAG TCCAGGGTGG ACACTCTGAC TGGGGAGGTC AATTTCTTGA   720
   AATATTTATT TTTGACGGTG AGTTAAGCCT TTATAAGAAC CTCCTTTCTT TTCTCACATC   780
   TCACAAGGAG TATGGGCTGT AAGAGGGGAG GCCTGAAACC CAACACTACC CACTAGGGAC   840
   TCATCTCCCC AGGTACCCCA ACTCTGTGGG CCTGGAGTCA GCCATCCTCT CCACCCCAAT   900
   CCTCAGAATC CCCAGGTCGG GGTAATGAAG ATGGAAGGCT GGGAGAATCC TGAGTTAGGT   960
   GGAGGCGAAT GTGTCCCTGG TTCATGGCTT CCAATCTGTC TGGGAAATCA CCCAGACATA  1020
   TAAGGGGCAA AACCAACCAG AAATCTTCAT TAATTCTGGG GAGTTGATGG AGCTGTTAGG  1080
   AACTCTGTGG GAGGTGACAG TGTGAGTCTC AAGGAGTGGA CTGACCTTAG TGATGGGGGA  1140
   TCAAACACTC CACCACCCGG CCCTCTTTTG CCTGTGTCTA ACTTGGGGGT ACGTGCTCTG  1200
   GGCCAGATGC TGTGTTAGAA GTTTATGTTA TGGGTATCTC CATTCTACAG ATGGGAAAAC  1260
   TGAGGCACTG AGGGGTTAAA TTACTTGCTT CATTACCTAG CTAGTCAATG GTACAGCCAA  1320
   GACTCAAAAG TGAGTCCAAG TGACTCCTTA ACTAGAGTCC ATCTACTGCC TCGGAGTACT  1380
   CATGTGGTTT CAAGGAAGAG GCATGCCTGC CAAGGAGCCC AGCTCACTAT GGTGGCCAAG  1440
   TCAGAGCAAG GCAGAGTGGC AGCTGCAGGA GAAGTGTGAT GGGGAGATGG TATCTGAACG  1500
   CTCCAGGTTT AGGCTCCTTC CTTCTCCCCT GGAAGGCAGT TAAGACTCTC CCTATTATCT  1560
   CTCATTGCAC ACAACAATTC CAAGAGCTTT TCCCAAGACT ACCTGGCCCA GGCTTCTGGC  1620
   TTCCCCCGAG AGCCTTGAGG GAGCAGCAGA GGAAAACTGA GGCCCCCAGA GGAGAATGGA  1680
   AGGAGTCAGC CTGTGCGCCA TGCCTCGCAG GAGCTGTCTC AGGTGCAGAC TCACATCAGC  1740
   GACACCAACG TCATCCTGTT CATGGACAAT AACCGTTCCC TGGACCTGGA CAGCATCATC  1800
   GATCGAGTGC GGACCCAGTA TGAACTGATT GCACAGAGGA GCAAGGACGA GGCCGAAAGC  1860
   CTGTACCAGA CCAAGGTGGG CGTGGCCCAG ATCTGGTGCC CAGAAAAACA GATTCTTCCC  1920
   AGAATTGTCT TTCTCTTATT GCATTGTCTT TCTCTTATTT CTGAAGTAAA ATGTGTTTGT  1980
   TATACAAATT CTAGAAATTA CATGTAAAGA TTACCCATCT CTCACTACCG CTATTAATAT  2040
   GTTAATATCT CTTCTACCAG TTCTTTGTCG CTATTAGGCT AGTGGAAAAG TGATTGCGGT  2100
   TCTCGCCATT AAAAGTAATG ACGAGAACTG CAATTACTTT TGCAAAAACC CAATAATGTT  2160
   TATTGAGAAC TCATATGTGT TAGGCACCTA GCAAAGTGCT TTACTTATTT ATTATTATTT  2220
   CATTCAGTCC TTACAACAAC CAATGAGGTA AGAATTTCGT TATCACCATT TTATAATAGA 2280
   TAGTAGTGTG TGACATTACT TAATTTCCCT AATGCCTTGT AGCTAGTAAA TGCAGAGCCA 2340
   GAGCTTAATT AAAATTGGTT TGTGTCTACA AACCCATTCC CCTCACCACT AGAATGATTT 2400
   TTATTCTTTT TTCATAATGG TATCTATTAA AATATATTTT TTTTACTTTT TTTTTTTTTT 2460
   GAGATGGAAA CTCACTCTAT CTCCCAGGCT GGAGTGCAGT GGCCTGATAT CAGTTCACTA 2520
   CAAACTCCGC CTCCTGGGTC CAAGTGATTC TCCTGCCTCA GCCTCCTGAG TAGCTGGGAT 2580
   TACAGGCACG TGCACTACAC CAAGCTAATT TTTGTATTTT TAGTAGTGGC AGGGTTTTGC 2640
   CATGTTGGCC AGGCTATACT TTCTCTTTTA CTTAACACAT ATGGATACTT TTCTGTGACA 2700
   CTAAATAACC TGCCGCATTT TTAACAGCCT GGTATTATAT TGTTAGACTA CACCCTCCCT 2760
   TATTAGATCA ACTCCTTTGT GGCTAAGTTG TTGGGCACAT CTTTGGTTAC TTCTTTTCCA 2820
   TAAACTGACC TGGATCTTTT TGGATGGTGA AGCCTCTGGT TGAAAGGGTG TGGCTGACAG 2880
   TCCAGTCACT AAATTCTGAA CAACTAGCAT TGATGACTGG CTTTGAGGAT GATCTGTGGC 2940
   CAACTCCAAT CCTGGCTGAC CTCTGTCCCA CGGTCCTGCA CAGTGTTCTG GGGTGGAATG 3000
   GATTTCGACA TTAGACTAGG AAGCCAGATG GCCAACAGTG AAAAATAGCA GAGTGTACCA 3060
   GATTCCCTTG CAAGTCGATG CTTCTCCTAC CCACTTCAGA GCCCTGTGCC TGGGGGGTGG 3120
   AGTTCTGACT AATGGGGCAA TACAGAGACA GAAACAGAGA TGGAGGGGAA ATGAGACTGA 3180
   ACGTGGAGCC AATGGAGGGC CTCTGAGGAC ATGAGGTCTG CTTGACTGCT AGGGAGATCA 3240
   TCCTGGAAAA GGGTGGGAAG CTATATGGTG GGTGGAAAGA GTGAGGGGGT CTCAGTGTGG 3300
   GTAAGGACCA ACGTGAAGGC TTAGATGTGT GAAAAGGTGG TAGAAGGGCA TCACAAAGCA 3360
   GGTTTGTCTG GCCTGGGATG AGAGTCTGCC CAGAGACTGG TGGGAATGCG GGAGGCTTGG 3420
   GATAGTGTGA GTGTGTGCAT GTATACATGT GTTTGCAGCC TGGGTGAGGG AGGTTTGGTA 3480
   TAGCTGTGAG TATGCATGTA GGGGTGACCA CAGTGCAAGG TGGGTGGGAA TCTCCCAGGG 3540
   GAGAGCAGCC CAGACCTACT CCTCCTGGAG GGGCTTGTGG TGGGCAGCAC ATGCTGACTA 3600
   TGATGCTCGC TTTGGCCCCA GTACCAGGAG CTCCAGATCA CGGCAGGGAG ACATGGAGAT 3660
   GACCTGAAGA ACAGCAAGAT GGAGATTGCA GAGCTCAACC GCACCGTCCA GAGGCTGCAG 3720
   GCAGAGATCA GCAACGTGAA GAAGCTGGTG GGACGGGTGC TTAGGGAGGG CTGACCAAAG 3780
   CCCTGCACCT CCTACAATGC CCTGCCAGAT CGAGCTCTGG AAACTTAACC ATTAAATGGT 3840
   CTCCAACTGT CTCTGGAGCA GATTGAACAG ATGCAGTCAC TCATTTCGGA TGCTGAGGAG 3900
   AGAGGCGAGC AGGCCCTCCA GGATGCGTGG CAGAAGCTGC AGGACCTGGA GGAGGCCCTG 3960
   CAGCAGTCCA AGGAGGAGCT GGCCCGGCTG CTGCGTGACT ACCAGGCCAT GCTGGGGGTC 4020
   AAGCTGTCCC TGGATGTGGA GATCGCCACC TACCGCCAGC TGCTGGAGGG CGAGGAGAGC 4080
   AGGTGGGTCT GGCAGCTGTG TTTCTGGGGC TAAGGCTTGA GATGCACCAT GAAGCTGTGG 4140
   GACTGGCTAT TTGGAGAAAA GATAAGCCCA CCTTTTTGGG AAGATTGGTA GCCAGGTGAG 4200
   CAGAAACATT CCAATTAGAG GCAGAGGCTG TGTGAATGGA CAAGCCTCTT CACACAGGGA 4260
   GAAGTCATTG TTATCATTCC TCCACCTCCA AGTAGAATGT CCTTATACCC CAATCCAAGC 4320
   CTCTGCAGCT GGTATTCACC CCCAATGCTA AAAGGCTTCA TGAAAACCCT GAAATTTCTC 4380
   TCTGCCCCAC TGGCTTCCTG ACCTCTGCTC ATGCACACAC ATTTCCCTAA GGCTTGGGGA 4440
   CACCTCTGAT CCAGATGTCT GTGGCCACAG CCTTCTCTCC TCAGGCCCTG TGGGTCTGGC 4500
   TGACCGTGTG CTTTGGTTTT ACAGGATGTC AGGAGAGCTG CAGAGCCATG TGAGCATCTG 4560
   TAAGTAGCAG ACCCAGGGGC AGAGAGAGGC TGGTGGTGCT GGGTGGAGGG AGGGCCAGGA 4620
   GGTGGCCAGC AGAGAACGGA AAGTCTGGCA TTTTAGCTTC CAGTCCTGTG CAATAGACAC 4680
   CAAAGTAAGC AAGTGTAATG CAAAGCCTGG AAGAATTCAT TTCAAATAAA TGGTTATGAT 4740
   TTCAGGTCTG CTTATCTTAA TTGTTATGAT GCCTTTTTAT TAAATGATGC CTAGGAGGAA 4800
   TCAGCAGCGG CTAGAACTCT TTAGGGTACA TATTCAATAA ACAATGTAAG TGTGTTGCTG 4860
   AGAGGAACCC TGGCATCCCT TTGTAGTATG AAGAATACTT TTCAAGTAGG AACACTTTCA 4920
   ATTTTCAATG TATCGGGTTT GCAAGTCGAT GCCACGGGTG ATCGAGGATG GAGGAGGCTG 4980
   CAGGTGCAGG GCGGGTGCAG GTGGGGGCCT TGCCTGTCCC TCTGACCCCG TGTGCACTGT 5040
   CCCTACCCAC AGCCGTGCAG AACAGCCAGG TGAGCCGTCA ACGGCGGCGC GGGAGGCGGC 5100
   GGCAGCTACG GCTCAGGAGG CTACGGCGGC GGCAGCGGTG GGGGCTATGG CGGCGGAAGA 5160
   AGCTACCGCG GAGGCGGGGC ACGAGGCGGG AGTGGAGGCG GTTATGGCAG CGGCTGCGGC 5220
   GGCGGTGGCG GGAGCTACGG AGGGAGCGGC AGAAGCGGCC GCGGATCCTC GCGCGTGCAG 5280
   ATCATCCAGA CCTCCACCAA CACCTCCCAC AGGCGGATCT TGGAGTAGAG GCCTCGTTTC 5340
   TGCCACACAT CACGCCTGCC CCTCACCGAC CTCTCCTCAA ACTCCTCCCC TCCACGCCCT 5400
   TCCTAATCCC CTCTCATTCA CTTTTCTTAA TGGGTCTCAG CAATTTTGCC AATAAATTCG 5460
   ACTCTAATGG GGGAAGCAGG GTGGATAAGT CCAAACAGCA GATCTCTCTT TTGGAGGGCA 5520
   CTGGCTTGCA GTCAGATTCA CAGCTAGGCA CATTCTCACT CAGACCCCGC TCTGCTGGCC 5580
   CTGCTGCTGT TCCTGCTCCC ACCTTTTTGG AAGATCGGTA GCCCAGGGTG AGCACAAACA 5640
   TTCCAGTTAG AGGCAGAGGC TGCGTGAGTT GGCAAGGTAG GGAGAAGTCA TTGTTATCAT 5700
   TCCTCTGCCT CCAAGTAGAA TGCCCTTATG CCCCAGTTCA AGCCACTGCA GCTAAGTATT 5760
   AACCCCCAGT GCTAAAAGAT ACCAGGCATC TAGTTTAGCA ATGGAGGGAA ACAGAAACAG 5820
   CCTGTAGAGA GCATCGACAA GGCGCATAAT GGAGAGTTGG TATCTCAACC CCAAGCTCCC 5880
   TTTGCTGTAC CTGGGCCTGC TCTGTGAACA AGAATCCACG CCCCCCTGCC CTGCTGGGAC 5940
   CACATAATGA TCCCTTTGGG GAAGTTGCTG ATTGCAGGGC AAGCTAGCTT GGTAAGGAAA 6000
   ACCTCTGCAC CAGCGGCCTA TTCCTGCCTC TTGGTCCATA GCCTCATACA CTCATGTTGA 6060
   TGGATAGTAT AGATTTGCTG CCCACACCAG ATATCTGTAA GGCATCACTG TCCTGATTCT 6120
   GAACCTCTGT TTCAGGAAGC ATTCTCCCCT GTGTAAACAA CTCAAGGTGG AAGTATTTCA 6180
   GAGGGCATAG GGTCATGAAT CCTTACCCAA AGGAAGCCTG TTTTAGCAGT GGATGCAGGA 6240
   GTTGATGAAC AGACAAGCAA GTTCTGCTTC TGTCCTGTTT CCTCCTGACA GCTCCATTCT 6300
   TTTGAAGCCT GACCCTTCCT AAGCTCTGCA TCATAACCAC TCTGAGAATT GCCCCATTGG 6360
   TGGGCATGTG AAGCCAGCTC TGTTCCATCC AGGTGCCTCG GGCCTGAGGA GTCTGAGGAT 6420
   CTGACTTGGG TCTTGGAAGG GTTCCAACCC AAGTCAGTCA GGAAGCTGCC CATTTTTTTG 6480
   CAAGGCATTT TAATGCCTTT CCCAGACCTC TCTAGTCCCT CCTGCCTTCT GTTCTCTCGA 6540
   CAGCTGTGAG CCCTTTAGAG AGAATATGAC TCTTAATTTT GAATCTTATG TAAGAGGCTT 6600
   GAGATGTGCT GGAGAGGGCA GGAAGAGGAA AGTATCAGGC CTTGAGAGAG GGAATGTAGC 6660
   TTTGCTTCTA TGATCTGGAG TCACCTTCAC TTGCTAGCTG AGTCCTAACA CAACTTCCAA 6720
   GTCCATGATT CTCTTGGGGC ATTGGATGGG CTCAGTGTGG GTCTCTTAGG CTGTTCTTGT 6780
   GACTTCATCA TTTCCTGGTT CAAAGTTGTA CTGTCAAGGG GCAGCATTTC TGGTATTTCT 6840
   ATAATAAATT TTCTGTGATC TCAAATTGCT GTTTGGTCAG GAGATGCATT ATTTCTTCTT 6900
   CTTCTTCTCC TTCTTCTCCT TTGCCTTCCT CCTCTTCTCC CTCCTCTGCT TCTCCTTGCT 6960
   CCTCTGGCTC CTCCTCCGCG CTCCTCCTCC TCCTCCTCCG CGCTCCTCCT CCGCGCGCTC 7020
   CTCCTCCTTC TTCTTCTTCT CCTTCTCCTT CTCATTCTTC CATCTTCATC TTCATCTTCT 7080
   CCTCTTTTTC TTCTTTCTTC TAAATAAAGA TGGGGTCTCA CCATGTTTCC AAGGCTGGTC 7140
   TTGAACTCCT GGGCTCAGGT GATCCTCCCA CCTTGGCTTC CCACATTGCT GGGATTACAG 7200
   GTGTGAGGTG TGGTACCTGG GCTATTTCTT TAAAAATTTC TGCAGACCTC TGAAATTATT 7260
   TATATTTGGG AAGTTAAAAT TTCTTCTTAT TTTTTATTGT ACAAGTAATA CACAGTCTTG 7320
   AAGAATCTTA CAGACATAAT CTTATTAATC CTTAAAGTGG CTGATCATCC AAAAGTCAAT 7380
   TATACATTTG TTCAATGAGC ACTTATTAAG CTCCTACTGT GTGGCGGGCA GTGGCTTAGG 7440
   CACTGGGAAT GCAATGTTGA ATGAACATGT TTCTGACTCT TAAGTTGCTC ACAACTAAAT 7500
   GACATATTAT GGGGGAGGGA CGATTCAAGG AGAGAAGAGA AATCTGAGTG TGCTTCTAAG 7560
   GACCTCTAGC CTGAGAGTGG AAGCAAGGCC TATCCTGAGG ACACAGGCAG ACCCCCCAAA 7620
   ACAGGAACAG GTGGGACTTA CGACAGGTGC CAGTGCTGGG GAAGGGACGT TTGGTTCCAA 7680
   CAGACTCCTG GAGGACTGGG ATATGGAACA GGGCCAAGGA AGAGAGGTGT GGGTGGGGAG 7740
   ATGAGGGAAG GGCCCTCCAA ACAGGGGGAT AGTCTGCTCA GAGACTCAAA ATAAGAGAGA 7800
   GTGTGGGGGT GAGAAGGAGC AGCTGGACAG GAGAAACTGA GCTAGGGAAG GAAGGGGCTG 7860
   AGGCCACAAA CTGAGTGGGG TCATGGGCAG AGACATCTTC AATTGATGCC TTGAGGGAAG 7920
   CAGAGATGCA GAAATTCCAT AATGGAGCAA GTTAAGCCAT CACCTCATCC TATGTGGTAG 7980
   TTCTCAGTCC ATGTAAAAGA ATCACATAAA AGATGTGATC TACTTTCTAA TTCCCTGGAG 8040
   GACTTTGCAT GCAAATTTGG ATATGGGATT CATTCGAATA TGACAGGAAC CCCATATTGA 8100
   TAAGACACTG TTGCTCCCGG GTGGGCATTG TTCAACTCAA GACTTGATGA CCCAGATAGG 8160
   TGTGTCTTTG CAGTTAGCTG TCACATGTCC CACCGTTGAA AGGTGGGCTT CTCCTCCACA 8220
   TGTGCAGGGC TCTCTGCCTG CCTTTCCCTT TTCTCGTGTC CTCTGACAGC CTGCTGCCAG 8280
   GATAGATGAG ATGGGGAGAA ACTTCTCAGA GAGAATAGAG GGGTGTGCAT GGAAACAGAG 8340
   TGTCTTATCA CTATGGGTTG ATATGATGTT TGCAGTTAGC TGCCACATCC TCCCCAAAGA 8400
   CTTCTGGAGG GCATGCCTGG GAACACAATG TTTTATTCAT ATGGGTTGCT GTCCTATTCC 8460
   AATGAATCCC ATATCCAAAT TCCATCAATA TCGCCTTCAG GAAGCTACAA CATATTCGGC 8520
   TCAATATAAG AAGCACCTTT CTATGATCCT GACATGGGAG AGGCTACCCT GGGGAGTGAT 8580
   CAAGTTTCAA GTCAGAGATT GGCTAACCGT TTGGCAGGAA CGTTGAGGGC GGGAGTGGAG 8640
   ATGGGTGGGG ATATGGTATG GAGGCATCTC ACTACTTTGC TGTACTAAGA GTTCACATGG 8700
   CGAAACCTGA GAAAAAAAAT TCTACTCTCT GTGTTATATG GGAAGAATAA GGTCAGGTGC 8760
   CAGTGAAAGC TAAAGTCACA AAGAAGCCAA AGGCCCTAGC CAGAACTGTT AAATGAGGCT 8820
   AAGTTTTCTG GCAGCACAGG GTCTATTACA GGGTGTGAGT TTGATTATCC CTGGGATCAT 8880
   GCATGTGTGA TACTCTAATG GGATCCACGT TGGCTCTGAG AAAACACGCA AGGATAAGGC 8940
   CAACCACAGC TCTCCTTTCC CATCCTCTCT TGGGAACAAG TTGAGATTGT CCCAGAAAAT 9000
   GTGGCCCTGA CTTATCTCTT CCGAATTCCT TGATTTTGTC CTGTCATGGA GGCCTGGGGG 9060
   ACAGATGGAG GGAATCATGT GCCTGAATCT GAAGAATATT GGAATAGAGA TTCCACAAGG 9120
   TAGGGGCAGG AGAAATAAAG GACAGAAAGG AGAGGAGTTG GTCAAAGAAG GCATCTCAAC 9180
   GTCTAAATGA GAAGTCTTAA TTCGATGTTC AGGGAAAGAA AGAGTAACTT TAGGGACCTA 9240
   AACAAGGAGG ACTAGCACTA AGACACTGAA GAGATTTCCT GAAATAGACA ATATTTCCAT 9300
   CAGAGACAAT GAGAAATCCC ATCAGGAGAA AATGTCTCTC ACTTTCAGCT CACCCCAGTG 9360
   AAAACAACAA GCATTCTATA AACCATGTAG GAAATGCCCA CACATGCATT ATCTCACCTG 9420
   AGTCCCACTC ACCTGGGAGT GCGGAGACCA GGTGTGGGGG TCTGCAGTCC TTCTAGGGAC 9480
   CATGGAGTGC TCCATCCCTG CCCCTAATCA ATGCTATTCC CACAAGGCAG ATACTCAGAG 9540
   GGAGAGCCAA GCAGGCTCAT TGCAGTGCAA TAAAGCCAAG AGGCTGGCAG GAGGGAGCAA 9600
   ACACCCGGGT TGGTGAGAGT CCCAGGGAAA GTCTGCCAGT CTGCTCTTTG CTCTGAGAGG 9660
   CAGGGTGGCA GGGTTGGGGC ACTCTGGAAA TATAAATTTA GTTCCACCAG CTTCTCATCC 9720
   ACAGAGATTT TGATCTGAGG ACATGGTTAA CTGGAGGAGC AATCATTGAC TCAGTAAAAT 9780
   TCTAACTGCA TCTGACCTTA GACAAGGTGT GCGTTTCTGG GCTGGGAAAG TTCCTGGTCT 9840
   GAGGAAGAGT CTCTTGAGAA TGTCATCTCT TTTCAATTAC CCAGCCTTTT GGCCCAGAAT 9900
   GCATCTTCAA ATTAATGAGC CATTTGCTGG TTAATTTGGT CCCAGGGAAA AAAGTCCAGC 9960
   AAATTACTGG GCATTACACT GAGCTTGAAG GTCCCTCTTC AAGGTTGCCC TGGTTTTATC 10020
   AGCTTTCCGA TCAGTCTGGG AAATGGGATG TCTTCAAGGC TGATCAATGC TCTGTTGAAG 10080
   GGCTGGCTGG GAATTTGGGG TATTGGGAGG TTTTCTAGCA TGGAGTACGG CTCCGAGTGG 10140
   CCCAATCCCA AGCCTGGAAG GGCTTCCAGG GGGCTCTAAG TGTGCATTCT GACCTCCACA 10200
   CCTGCCCCTG TGTGCTCAGC CCTCAGTGTT TGTGCTCCCC CTGCAGAGCA GCTCTGCAGT 10260
   GAGGGCAAAG GCTCCTCGCA TCTGGCCCCA GCTCCCTCCA GCCTCAGGTG AGCCCGGTGA 10320
   TGCACCTGTG ATCTCTTCTT CCATGTGATG CCCCCTAGCT TTCCCAGGGC AAGTCCGTGG 10380
   ACTTCTTAAG GCTTTCTCTC ACAAGATGAG GAAATGGGCC CATGTCAAGG GCTTAAATGT 10440
   CCTGTTCCAG CCTTTTCACT GTTTCCAGTA AATCAGGGGC TTGTTCTAAA GTTTGTTTTT 10500
   TTTTTTCTGG TTATTATATC AGCTTCTGGG TTCTCTCAAA TGCAAGAGTG AGGGAAAATC 10560
   TTCCTTTTTT CCTTTTTTGA GATGGAGTCT TCAGCATCAG TAGCCCAGGC TGGAGTTCAG 10620
   TGGCGAGATC TCGGCTCACT GCAAGCTCCG TCTCCTGGGT TCACGCCATT CTCCTGCCTC 10680
   AGCCTCCCGA GTAGCTGGGA CTACAGGGCC TGCCACCACA CTGGGCTAAT TTTTTTTGTA 10740
   TTTTTAGTGG AGACGGGGTT TCACCTTGTT AGCCAGGATG GTCTCGATCT CCTGGTCTCG 10800
   TGATCCGCCC ACCTCTACCT CCCAAAGTGC TGGGATTAAC GTGAGCCACC ACGCCTGGCT 10860
   CCTTTTTTCT TTATCTATAC TCTACTATGC TTCAGTTTCC CTGGAAGGTA CATAGAGCCT 10920
   CCTTTTACAG AGAGAACTAG CTCAGAGAGG TCAGTGACCT GCCTAGAGCA GTGCAGAATC 10980
   AGGAGCGGAG CCCAGCCTGG CAGCCTCCAT GGCACAGAGC AAGATGGGCC CCACCGCCTC 11040
   TCTCCTCCAT GTTCATCTTT GGTTTCCTCC TTCCTGGCCT CTGCTCTGCT CCAGCCTTGC 11100
   TAGTGAGTGA CTCCTGAGGA CCTCCTTCTT TGCTGTCCAT CCTAAATAGG GCTGCCCCTC 11160
   TGTCTGCAGC TCTCCCTCCT GCATAAGAAG CCTTGCGCCC TCCTCTGCTG CCTGGCTGCT 11220
   TTCAACATCT CGCCCCGCCT CCCCATTGTC TGTGATTTCT CTTCACTCCA CCCAGGCCTC 11280
   AATTTTCCTC ACTCCCCTGG GATTTCCCTG TCCCATGTCC CTGGTGGAGT CCCTCAGGGT 11340
   GGGTGGTTGT CATGCAGTGC TTTCTTCACT CTTTTCTTGG TCCCATCCCA CAAAAGCTCT 11400
   CAAAACATCA CCACACCTGC TCCTGCCCAT GCCCCACAGC CACCCCTGGC AGCCTCATCT 11460
   CAATGATCAG TTCTGGGTTG TGTGTGTGAG TCCTTGGGTG GGGGTGTTTT GGTGCTCTGT 11520
   CATCAGCACC GCTGGGGTAA CTCTCAAGTA TAAGGGGCCA TGTGGGATGC TGGGAGGGCA 11580
   TCAAAAGACA CAGGGGACTT AGTCTTGCTT TCCAAAGGCT TCCAGAGTGA TTGAGGGGCC 11640
   AGGAAACACA CAAGCACATG CATGAAAATG AGCCAACAAA TGCATCAATA TGTACTAAGT 11700
   CTGGCAGCAG CCGAGCTTGG AAAAAGAGAC CAATAGAGCA CTTGCCCGAT GTGGACTGAG 11760
   CAAAACTCCC TGGAGGAGAT GAGATCTGGA CCTGTCTGCT GCCTGCTTTG AGTGAGAGAT 11820
   GAAGGCATTT GCCCACAAGC CCTGATGGAC CAAAAACAGA TTCAGGACCA AATGCTCAGC 11880
   CATTGAGATC TTTGGTGCCC CAGAGCTTGA CTATGGGTAG GGATTTGTGG CAATGCCGAG 11940
   GCAACCAGAA GACCTTTCAG AAAAGAGAAG AGTAGAAGTG GGCTTGGAAG ACAGAGAGGA 12000
   ACAGGGATGG AAAGGGAAGA AGAGGGTGAT CAGTCTTGGG CAAAGCACGA GAGCTGAAGG 12060
   GGTCAAGGCT GTGAGGCCGG GGAAGTGGGT GAGCAGGGTA AGATGTAGGT GGTGCTGGTG 12120
   GTGAGAGCAG GCCAATGACA GAAGGAGCCC ATGTGATGCG GCGGGCTTGG ACTCTGGAGT 12180
   GAGGCACGTG GCTTGTCAGT TACCTGCTGG ATGACTTTGG GCACATATTT CAACCTCTAT 12240
   AAACCTAAGA TGCCTTTTCT TTAAAATGGG GCTAAGAGCT CCCACGACGC AGACTTTGTG 12300
   TGGTATTTAA ATGCAATGTG GCTCCTAACA GCATAGTTGC TGCGTGTAGA TGTTAGTGTC 12360
   TCTTTCTTTC TCATTTTGTC TTTATTTCAT AAATGCACAG TCACTAAGTA AGAAAGGAGA 12420
   GAGTGTGTGG CTCACACTTT CCTGCATGTG GTTCTTCATA TCCCACACAC CACACTGATC 12480
   CTGGGGACAT CACAGGAGAT GACGGGCCTG GTCTGGCAGC ACTGCAGCTC CAGCTCTGTT 12540
   GGGCTGCCTC GAAAGTGGGC AGTGGAAAAA GAAAAGGAGT TTGATTCAAC AATTGGAAGA 12600
   GTCTCAGGAA TTGACTTATG ACTTGGACAC TTTTTTTTTT TTTTTTTTTT TTTTTTTTTT 12660
   TACTTTTTTG GGCCTGTGCT CTCACTTCTC TGTGAGGCAG GTTAGATGAT GTGACCTTTG 12720
   AGGCCCCATG GATGAGAACA TTCTGTAATT CTCTGTGTAC TTGTTTATAG GGCCCAGTTC 12780
   CACTTGCCTG TCTTTGAGCC TCTTCCCGGT TCAGGGAGGA ATGTCACTTG AATTGAAATC 12840
   AGAAAACCCA GATTCTGCTT CCAGATGTGT CTTTTCCTAG CCGGAGTGTC TAGAGGAAGC 12900
   CACTTAATCT CTGAGAATCA GTTTTCTGTT TCATGAAATG GGTTGAGAAC AGCTTGATTG 12960
   CCTAGTTCTC AGGGCTCTTG TGGGATGCTC TTTGCATATG TGTTTGGTGG GGTGAGCTGT 13020
   GCAAATGTAA GCTATGGTGA GGTTTATGGC ACTTATTCCT GCTAGTCCTG CATTTCTCCC 13080
   TTCTCACAGG AGCACCTGGG GTATGTTTTG CAGCTAAGTT GTCTACCAAT TCCCTGACCA 13140
   TTCATTCAAA CCTTTGATTT TTCTGTATGT CAGTTTCTTA GTTCAAAGAT GGGAGTGTGG 13200
   ATCACTGCCA AGGTCTGTTT TTGGCTGGCA CACACATGCA CACAAACATG TGTGCACACA 13260
   AACATGTGTG CCCAAACATA CTCACACCCC TCCAAAATGC TAGAAGGAAT CGATTGTGCA 13320
   GAACAATATG TCTCATGAGG GAGTATGCTG AACTAAAATA ATTTTGATTG CTTGTCAGAA 13380
   AATGATTAGG CAACAGTCAT TACCATGCCA AGACTGTCCC AGTCTCCATT GTTCCTAACA 13440
   AGACCTGAAT TACTCATTCC CTAAAGAGAT GGTTGGTTTA GCAGCCGAAG GATTTTAGTG 13500
   CTAGACAGAG TCCCAGACAG CAGTGCCACA GTGATGGCGA GGGAGAGGAG TAGCAGGGGA 13560
   GCGGTGAGGG GCACTTTCTG GAGGAGGGTA TAGGGCAAAA ACTGGGAGGA GAAGAGGGAC 13620
   AAGGTTCAAT AGCGGAGTGC AATGGAGAGG ACCGACACAG CCAGCCCGAT TCAGAGCCAC 13680
   AGAGTAATGG GACCAGATGA TCTTCACAGA CTCCCTTTCT CCCATAGATC TTGCACACCA 13740
   TAGTGGAGAC TTCCCATGTA CATCTATGGT TTGCCACTTA CAGAGTTACT TGGAGCCAGC 13800
   TGAAGTTAGA GCTGGCTTCT CCCCTTTGAG TCTTCAATTC TGTGTTTATG TGCAGGCCCG 13860
   GGGACCATGC CAGGCTTCTA AGAAGGTCTT CGAATGAAAG TCTGCTTGGG CTCTAGTGTG 13920
   TCCAGATCTC AGTGCCACTA TTATCCACTG ATATTGATCA AGTGCTGCTC TCCAGGAAGA 13980
   CCCCTGAGGT TTCCTGGTCC ATTGCAATGC ATGCTGGGTA CTCTTGCACT TGGATGGAAG 14040
   TAAAATCTCC TCACTAAACT CTGTGCCACC AAAATCTCCT TCTCAGTGTG AATTGAAGAA 14100
   ACATTTTCCA AGACTTGCAT GTGCCAGGAG CCAAGGACTC AGAGTGATAA AACAGCCTTC 14160
   TGCCCTCAGA GCTCTCTGTG GTGGGGCGCT TCCTGTGCTG TCTGGCTTTA CACACAGCAG 14220
   GCAGAATGAC TTGAATTCGG CTGCTGTGCT GTCGTACAAC ATGCTGTTTA GGATCTTGCA 14280
   CATGATAGCT AGGTATTCTT GCTTCAAATC GCAGGCACCC CACTTACCAA CTGTGTAGAC 14340
   TTGATCACGT TATTCAACCC CTGTGTCTCT GCTTCCTCAT TTTACAAATG GGGAGAAAAA 14400
   TAGCATCTAT CTCAAAGTTG TGAAAATTAA GCAAGTTAAT ACATATGTGC TACGTAGAAC 14460
   AGTGCCTGGT ACATGGTCAG TTTTTGATAC ATGTTAGGTA TTATCATTAT TATCACCTCC 14520
   AGAAACAATT TAAACTTCTC ATATAAGGCT CTCCAGACAC CTCTCATTGT CTTCCCTTCC 14580
   AAATCTGCAT TTATCTCTCT CTCTTTGCAG TCCAGTGTGA GGCTTGAATC ACCTATCAAG 14640
   CCTCACCTCC ACCCCTGTGC TTTACAAAAT GTCCTAGAGC TTCTATTTAC TCGTCTCACT 14700
   GCTCTGTGGG CTTTTTCACT CAAGGGCGTT TGCATGCTAT CCATTGCTAC CTGTTTTCTG 14760
   TTGCTGGTGT CTGTCTCCTG CTCTATCTTT GAAGAAAAGA AACAAGAAAA GGAATAACTG 14820
   AGAAACAGAG AAAAAAAATG TCTCTCCCTT CTGGTTCTTC CAGACCACCC ACTCATCCAT 14880
   CTTGTTCAAT GACAGCTTCT CTTCCTTTAA TTAATCACTG TGGTATATTT ATAAAGCTTA 14940
   TATTTATGAA AGACCTTTTA ATTTTTTAGT TATTAAAGCC CTTTCTCTTT GTCAGGTTGT 15000
   AACTGAGTGA GCTCTGGAGT TTGGAAAGAA GATCTTAGAA ATGGGCCAGA GAGCTCCTTC 15060
   TGAGATCCAA GCACGGAGAA TTGCACCTGC TGTGCATGGT AAGAGAGTGT GCTTGGTAGC 15120
   TCACAAGGGC AAGGTGAGAA TAGAAACTTT CATGCCTTTT TGATGGGGGT TATGAAATCC 15180
   TACCAAGAAA CACCAGGTAT CAGATGTGGG GTCCTGTTTT CCCAAAGCCA CAAATGCTTG 15240
   AAGGAAGATC TTGTGTGATA AAATAATTAC CACATGAACC AATCTTGCAT GCACAGCAAT 15300
   TTTGAGAGCC CATCCTGGGA GCTAGGTGTG TAGTGTTTAT CGTATTGTTG AGGCTCGTAA 15360
   AAATCTTGTA TGGCTGCAGG CAAGCCAAAC CCTTGACAGG CACTGCATCT CCGCTGACTC 15420
   TAGAAGACCA AGCCCAATTT CTTCCCTGTA TATAAGGGGA AGTCTCTATG CTTGGGGTAG 15480
   AGGAGTGTTT AGCTCCTTCC CTTACTCTAC CTTGCTCCTA CTTTTCTCTA AGTCAACATG 15540
   AGTCGACAGT TTAGTTCCAG GTCTGGGTAC CGAAGTGGAG GGGGCTTCAG CTCTGGCTCT 15600
   GCTGGGATCA TCAACTACCA GCGCAGGACC ACCAGCAGCT CCACACGCCG CAGTGGAGGA 15660
   GGTGGTGGGA GATTTTCAAG CTGTGGTGGT GGTGGTGGTA GCTTTGGTGC TGGTGGTGGA 15720
   TTTGGAAGTC GGAGTCTTGT TAACCTTGGT GGCAGTAAAA GCATCTCCAT AAGTGTGGCT 15780
   AGAGGAGGTG GACGTGGTAG TGGCTTTGGT GGTGGTTATG GTGGTGGTGG CTTTGGTGGT 15840
   GGTGGCTTTG GTGGTGGTGG CTTTGGTGGA GGTGGCATTG GGGGTGGTGG CTTTGGTGGT 15900
   TTTGGCAGTG GTGGTGGTGG TTTTGGTGGA GGTGGCTTTG GGGGTGGTGG ATATGGGGGT 15960
   GGTTATGGTC CTGTCTGCCC TCCTGGTGGC ATACAAGAAG TCACTATCAA CCAGAGCCTT 16020
   CTTCAGCCCC TCAATGTGGA GATTGACCCT GAGATCCAAA AGGTGAAGTC TCGAGAAAGG 16080
   GAGCAAATCA AGTCACTCAA CAACCAATTT GCCTCCTTCA TTGACAAGGT GAGTTTCTCT 16140
   CTCATTGCAC TGGTAGGGCT GCCGCTGGTC CACTTGGGAT TGGTGCAGTC AAAACACATG 16200
   TAGGTTTGAA CCTCAAGTTT CCATGTTTAC ATGATTAAAA GGATGTTTTG TGGAATGGTC 16260
   TCCTAGGAGA TATGTTAGAT GTATGCTTGT GAATGGTGTT AATGACTCTC TCTTTGACAA 16320
   AGGGTTCGTG GTCGACCTAA AGGTGGGTCA GTGTGACATT AACATTTAAG TGCTTTTTAT 16380
   TCAGCTCTTG AGCGGAATTG GGACTCATAT CTGTTGAATG AAGATAATAG AAATGGGGCT 16440
   AACTGAACTT TCCAGGGTGC AAGTGAGAAC CCTGGAAAGG TCTTCCTAAC CATAGAAAGG 16500
   GAGTTGAGTG TGAACATAGT ATAGAGTGTT ATTGTAGCAG AAAACATGTG GTCAGTCAGT 16560
   GCCAAACATC TTTTGCTGTC AGAGGGGAGC TCTGCCTTCT AATAATTTTA CATTGGTACT 16620
   GGATGAGGCT AGAGTTTTTT TATACTAATA TCTCCAAAAA TCAGCTCTAA AAAACTCAGA 16680
   TAAACCATTT TTTTAATTTT TTGCTTAATC ATTAATAGTG CCAATCCAAG GTTATCCACA 16740
   ACAAATTTCA AATCCAATTT TGAATTTTCC TGATATACTT TTGAAATGTG TGTGTGTCCT 16800
   GGGGATGCAA ACCAGTTTTT ATGGTAATAT ACCTAACAAA ATTTTGGAAG GCAAATCTCT 16860
   TAAATACCAT GCACCTATTT CAAAACATAA TTGCAATAAT TCTGTATGCG CTTTGCTATT 16920
   GGTATTTGTT TAGTTACTCC CTTCCAAGCC CTCTCTGAAT TAACAAGTTG GGTTTTATTA 16980
   TGCAGATGAT ATTAACTTGA TCATCTTCTT CCTATTTCTC TGTCATGGTC AGAAGATAGG 17040
   AATTGAGGTT CTTTTCCAAA TGAGGCACAG TTCTCCATGG CTATGAGACT CCATTTATGC 17100
   ATCAGGAGTA AAGGGGTCTT GTGTTTTTAG GTGAGGTTCC TGGAGCAGCA GAACCAGGTA 17160
   CTGCAAACAA AATGGGAGCT GCTGCAGCAG GTAGATACCT CCACTAGAAC CCATAATTTA 17220
   GAGCCCTACT TTGAGTCATT CATCAACAAT CTCCGAAGGA GAGTGGACCA ACTGAAGAGT 17280
   GATCAATCTC GGTTGGATTC GGAACTGAAG AACATGCAGG ACATGGTGGA GGATTACCGG 17340
   AACAAGTAAG GGACCCTGTC TGGGCAGTTC TTAACTTTTG CTGTAAAAGA GTTCCAGAAA 17400
   GTAATAAGCT AAGATCATGA AGCAGCATGT AGCTATGTCT TTTCTTAGGT TAGAGGCACA 17460
   TCAGTTTGAC ATTTTCAGAA ATCTTCATTT TCTCAGGAGA TGGAAATAGT CTAGTGGTTT 17520
   TATTGCTCAG TAGAAAGTAG TGGCCAATAT GTCCTAGGTT CATAATAGAA AGGCAGTGAT 17580
   AGGCAATGCC ACCTTTAGTT TAGAATGCTG GACTTCAGGT CTTACCACCT CTGAATCTCC 17640
   TAATTGTTTC TGCTTTCCTG CAGGTATGAG GATGAAATCA ACAAGCGGAC AAATGCAGAG 17700
   AATGAATTTG TGACCATCAA GAAGGTAAGC AAATTCTGTA GGACGGAACT CACATTTGAA 17760
   ATAAATAAGG GAAGAGGGTC TCCAATTACT AAGCAGAAAG CAGCCATGAT ATGGAGAGCC 17820
   AGGTAGTAGA CCTGGGGAGT ATATGGAGTG GGGCTATATT TTTCACATCA TCATGGACCT 17880
   GGACTGATCC AGGCACTTGG CTTCTCCATA TTTCCCAGCA CCTTACATAG TAAGTGGAGT 17940
   GGCAGATTCT CAGCAAGCCA GGCACACTCC CTTGATGGTG CTATCCGGGG GTGGGACAGT 18000
   TAGGGAACTG TGATTTACCT GGGGCAAAAA GGAGTGGAGT AGACCCAAAG CTCCTTTTTT 18060
   TGCTTGGAGA ATCCCCTCAC AGGTAATGAG AGGGACCTGC CCTGGAGAGA ACGTGCCTTC 18120
   ATGATGTCCC TTGTTCCTCT AGGATGTGGA TGGTGCTTAT ATGACCAAGG TGGACCTTCA 18180
   GGCCAAACTT GACAACTTGC AGCAGGAAAT TGATTTCCTT ACAGCACTCT ACCAAGCAGT 18240
   AAGTCTTCCA GTTTCAACCA AGTTTATCTA AATGGAGAGT TTTTAAGCCG GAACCCACAA 18300
   CGATTCAGAA GAATAGATAT TTATCTTTTA TTTCCTGACT GCTTTCTCTG TCTAAGTTGT 18360
   TTTTTGTTTT AGTGCTGTAA GAGTCACTAA CCTATTATGT CTTGCAGGAG TTGTCTCAGA 18420
   TGCAGACTCA AATCAGTGAA ACTAATGTCA TCCTCTCTAT GGACAACAAC CGCAGTCTCG 18480
   ACCTGGACAG CATCATTGCT GAGGTCAAGG CCCAGTACGA GGATATAGCC CAGAAGAGCA 18540
   AAGCTGAGGC CGAGTCCTTG TACCAGAGCA AGGTGAGTGG GCTGAAACCG TAGCCAGTTT 18600
   CCCTGAAATG GCTTGTCTTG CTATCCTGTG TTATCTCATG TATGTGTGCC TGTGCCATGC 18660
   TGAGTTCTGC CTACATTTAA CAAACGCTAT CTACCATCTT TAGTATGAAG AGCTGCAGAT 18720
   CACTGCTGGC AGACATGGGG ATAGTGTGAG AAATTCAAAG ATAGAAATTT CTGAGCTGAA 18780
   TCGTGTGATC CAGAGACTTA GATCTGAAAT CGACAATGTC AAGAAGCAGG TATGTGCTTT 18840
   CTCCTTCTAC CACTCAGCTG TATGGAATGG GGGTAACCCT CAGGTAAAGG GCGAGTGCTT 18900
   TCCTAGTTTT GAATCTTGCA ATTCAGCCCA AGGCTACATT ATTAGCCCTG GTTCCTTTTC 18960
   TGACTATGCT AGTTTCCAGA ATGCAGCCAT CATGCTGGGT TCTCTTTAGG GAAATCTGTG 19020
   AGAATGGCCT AGTAGAGAAA GATGGGATGG TCAATGTGAG TGATCTAGCC TATGACCCAA 19080
   AGTGGACTTA AGAGTTGGGG AGTGAGAGGA AGGGCAGCCA GGAGGTTTTA GAGTAGGTGT 19140
   TTAGAAGAAT GTCAAGTCTG TAAGGGTTGT AGGAGCCTTG ACTCAGGGCC AAGAGAGGCT 19200
   GTTGAGTTAT CCCTAAGGTC TTTTAAGGAA GTCAACATGG TGATGTGTTA TCTGGAGGTG 19260
   GGTGTGAGAT GACTTAAGGC CAAGTGGTTC TGTTGGACTC ATTATTGGCC TCACTGGAGT 19320
   GGGGAGACCA ATTGGGATGA GGAGGCCTAG TGGGGAATGC ATATTATGAG AGGGTGTCAT 19380
   ATCTTTTTCA GATCTCCAAC TTGCAGCAGT CCATCAGTGA TGCAGAGCAG CGTGGCGAGA 19440
   ATGCCCTCAA GGATGCCAAG AACAAGCTGA ATGACCTGGA GGATGCCCTG CAGCAGGCCA 19500
   AGGAAGACCT GGCCCGCCTG CTGCGTGACT ACCAGGAGCT GATGAACACC AAGCTGGCCC 19560
   TGGATCTGGA GATTGCCACC TACAGGACCC TCCTGGAGGG AGAAGAAAGC AGGTGAGGAA 19620
   GGGACGCTGG GAGTCGAACC TCTTCTCATG GTCTTCCTTC CTTGCAAGCT GATTGTTGTT 19680
   GAAGATGCAG CCATCTGATT GCAGCTTGTG CTGGGTATGG GGAAATGAAA AGTACACGGA 19740
   GCAGGAGGAA GGACCTAGTT TTACTTTGGG AGCTGGAGTC CCAAGCTGTT TATTTTTTTC 19800
   TTCTAGGGCT GTAACATATC TAGAAAGAGC TTTGAGGTGG AGCAAATTAT TCTTTATCTG 19860
   GGCTGCCTCA GATGGCAGCT GGCCTAAAGT CGGCATCTTT AGAGGGGGCC TTCATTGGCT 19920
   GCAAGGCTCG TCTCGTTTAT ATGGGAATTT CTCCGTGTTT GTACTCTTGC TGAGAAAAAA 19980
   TGACAGGTCT GGGAGGCCAG AGGGGATTGG ATTAAGTTTC AGATTAAGTG CATTGGAGAA 20040
   GACCCAGATG GGGAAAGTCT TCAAGGTGGT GGAGCGGGGA ATGGGGAAGC GGTTTGGGAA 20100
   GCTGGAGTGT CCTGAGGAAT TTTCTTATTT TCTCCTACAG GATGTCTGGA GAATGTGCCC 20160
   CGAACGTGAG TGTGTGTAAG TACAAGTCGA TTTCTCAGGG GCATGTGCAG GCTTTGTTGG 20220
   GCTGGAAACG GAGTTGAGGT TGAAAATAAC TGAGCTTCCT CTTGCAGCTG TGAGCACAAG 20280
   CCACACCACC ATCAGTGGAG GTGGCAGCCG AGGAGGTGGC GGCGGTGGCT ACGGCTCTGG 20340
   AGGTAGCAGC TATGGCTCCG GAGGTGGTAG CTATGGTTCT GGAGGTGGCG GCGGCGGCGG 20400
   CCGTGGCAGC TATGGCTCCG GAGGTGGCAG CTATGGCTCT GGAGGTGGCG GCGGCGGCCA 20460
   TGGCAGCTAC GGCTCCGGAA GCAGCAGTGG GGGCTACAGA GGTGGCTCTG GAGGCGGCGG 20520
   CGGCGGCAGC TCTGGCGGCC GGGGCTCTGG CGGCGGGAGC TCTGGAGGCT CCATAGGAGG 20580
   CCGGGGATCC AGCTCTGGGG GTGTCAAGTC CTCTGGTGGC AGTTCCAGCG TGAAGTTTGT 20640
   TTCTACCACT TATTCCGGAG TAACCAGATA AAGAGATGCC CTCTGTTTCA TTAGCTCTAG 20700
   TTCTCCCCCA GCATCACTAA CAAATATGCT TGGCAAGACC GAGGTCGATT TGTCCCAGCC 20760
   TTACCGGAGA AAAGAGCTAT GGTTAGTTAC ACTAGCTCAT CCTATTCCCC CAGCTCTTTC 20820
   TTTTCTGCTG TTTCCCAATG AAGTTTTCAG ATCAGTGGCA ATCTCAGTCC CCTGGCTATG 20880
   ACCCTGCTTT GTTCTTTCCC TGAGAAACAG TTCAGCAGTG ACCACCACCC ACATGACATT 20940
   TCAAAGCACC TCCTTAAGCC AGCCAGAGTA GGACCAGTTA GACCCAGGGT GTGGACAGCT 21000
   CCTTAGCATC TTATCTCTGT GCTGTTTTGG TTTTGTACAT AAGGTGTAAG CAAGTTGTTT 21060
   TTCTTTTGTG GAGAGGTCTT AAACTCCCCA TTTCCTTGTT TTGCTGCAAT AAACTGCATT 21120
   TGAAATTCTC CATGTCTCGA TCGCCCTTGT TTACGGCACT GTCTAACCTG GATGGGTGTT 21180
   TTGTGAGGTA AAAGAAGACA CTAGAGCCAC ATGGCATATG GGAAAGTCAT GCACACAAAC 21240
   ATGAGAAAAA TGCAGAGGCC AACCAGGCAA CATTTCACCA GACTGGAATC ACAGAGAGAG 21300
   CAAGCACTTT CCCAGATGGT GGGGATGTCA TGGAGAAATG GAGAGACCGG GTGACAGGTT 21360
   TTGTTCATTT GAGAAGGCTT TCTTGAAAAG GGCAGTGAGC AAGCAGGTTG GGAGGAAGAG 21420
   GTGTGGCATT GAGAAGAAGG GAAAGTATTG CATGAAAAAG TAATTCTTCA CGTGGAACAG 21480
   CCAGTAAGGA GGGGCATGAG TAATATAGGG TCAGCAGTTA CTGGAGCCAG AATACAGACT 21540
   TTGGCCTGGG GAGTTCAAGA ACTAAGAGTG GTAATAGAGA GTTGGATATT CCATTTCCCT 21600
   TCTCTTTTTG TGCCACCACC CAAAGCTCTG CATAATCTAA GAAGTTCCCT TGTTGACACA 21660
   TAGCTCATAC TTGTGAAGTT GTACAACAGG ATAGCATAGT GGCCAGAAGC ATGGACAGTT 21720
   GAACTCAGAT ATGCTTGGGT TTGAATCTTA CCATCACCAT TTACTAGTTC TGTAATACAG 21780
   TGCAAGTTAC AGACATCTCT GCACCTCAGT TTTAGTATGT CTAAATTGGG GATGATAATG 21840
   CCTTCCTTGT GGGGATAGTG TGAGGATTGA ATAAGATGAA TACACATGGC TGAGCACACA 21900
   GCAAGCACTA AATAAGTGCC AGTTTTAATG ATAACGGTGA TGATGATGAT GATGATGATG 21960
   ATGACGTAAC ATTGCTTGTG GGACTCCATA CAGCTCAGTA GATGCTTGCT CAAAGAAGCA 22020
   AGTTACCAAA ATTTTTGTAA TGGTTCTATG AACGTGAAAA AAGCAGTCAA CTTCTCTGAG 22080
   GATCAATTTC CTTAGTTTCC AATTAGGAAA AGTCTTCTTA GCTCCAGAGT CCCACAGGGC 22140
   TAATGGAATA AGGAGAGGAT AGATCACACA TGTATTATGC AAACACAACT CAGGTGAGCT 22200
   CTATTCTTCC TTCTCAGTTA TCCCTTCTGT AGGGACCCCA GTGTCCCCTG CTGTCTTTCT 22260
   GTGTCCTGAC CGGGAAACAC AGTGTGCCTT GTCTACTCCA TCACTTGGCC AGCTGCATGC 22320
   TTTCCTTTGC AGGCTTGAAG CAAAGCTGGG TCTCGGACAT TCTCAGGCAC TGACAAAGCT 22380
   GTTTAGTTGT TGCTGGGAAA CACTGGGAAA TAGCCCTTTT GTTAAACACA CAGAAACTAG 22440
   CCTTCGCCCT GAGCCAAATT CCTTAAACTC GTCTATGAAA TTCCATAACC TGACTCCTTA 22500
   ACTGCAGACA TACCCAGCTA GAACATCCCT CATGTCCCTG TCCACCGTGA GAATGCTGCA 22560
   CTTCACTCTG AACCTTTAGT CCTCCTTTTA AATACTGCAC ACTGATCACC CTGGTGTTTA 22620
   GTGCTTTGTT TTTTGGAATC CCACCTGGCT CCATTTTGGG ATGGTTCCGG GCACTTCCCT 22680
   ATGGAAATTC CCCTGCTGTC ACTGTCAGAG TGAGTCCAGC AGTGGGTTTA GCTGGATGAA 22740
   ACACCACCAT GTCCATTTCC ATTCAGACTA ATGTCAGAAT TTGAAAGGCA CTATGGTAGA 22800
   GTAGAAAGAA CAAGGAACTG TACTATTTAA AGGGCAGGCA AAGAAAAGGC ATCTATAGCT 22860
   TATAAGATGT GTGGATCTTT GGATGTGACT TGGCCATCCT GAGCCTAAGT TGTCTTGTAG 22920
   GAGAAATGGG AATGAGAATA TTTTCCTCTA GACATCAAGA GGAAAAGAAA TATAACGTGA 22980
   AAACCTTTGT GAATTGTGAA TGTGTTATAC AGAGTAGCTA AAAGAATTAA AAAGGGAGTG 23040
   ACAAAAAAGT AAAAGGCAGC TGGCTGCTCA GGGCCTCCAT GGAGGGAAGT ACCTTGATAT 23100
   GGTCACTGTG GCTCAGTGAC AGCTCTGCAG GGACAGGAAA TTGATTTGTT AGTGCACCCA 23160
   AAGTTGAATC TGCTCCTGAG TACTGATTTA TGGGAACCAA ACACACAAGA GATGAAGGAT 23220
   GTGTCAACCA GAATGTCCAG CATTAGCTTG TGGGGAAACA CATACTTCCA GTGACTGAAA 23280
   TACCATCCTG TTATCAAGAG ATCTGGGAAA CTAAAGTACT GACAAGAGCT GGCTTGATCT 23340
   GTGGATTTAG AACAATGAGA GTTAGGTGGC CTTGAGGGAG ATGATTCACT CTCCTTCACA 23400
   GAAGAGCTGA CCTCTGGGGT CAACAGATAT AGCACCTCTT TCCCAGGGAC GCTACTGAAT 23460
   GAACAGTGAT GTGTTCTTAT ACTCTGGCCC AGATTTTCTA CATACTTTCT TAGGTTACAA 23520
   CTTTATTTAG TCACATTTCA GTACTGGGGA TACTCCTGTT TATCTTCTTT GGACTCGAGT 23580
   TTTTATGGGA AGGTCATGAA ACAGAGAAAA ATACAATTTG CAGGGAAACT TACCAAGGCT 23640
   TGTAAGGTTA CAAGGATTAA ATGAAAACCC TGTGTAAGTC AGTATATAGT GAAGAAGTAA 23700
   ATTGAGTTAG ACCAAACGCC AAAATGCATC CGCATTAGAA AGACGATAAA GGAAGACTCT 23760
   GGATTCAGTT CTGTTCAAAA AACATTTTCT GCACAAATAC TATGTATGAG GAACTGGGCG 23820
   TTGGGGAGAT GATGATGAGT GAGACATGGT TCTTGCTTTC AGAGAGCCTA GAGACCTGGG 23880
   TGGTAGCAAT GGTAGAGATA CATCCAAGAC ACAGAAATAG ATATACAGGA ACACAGATGA 23940
   TTGAAAGTGA TGCTTGGCAG GGCTTTAAAG AATGAATCAG AGTTTTTCAG GCAGACGAGG 24000
   ATCTTCAAGG CAGAGGGAAT CATATAGATA AGGACATAGA AGAGTGAAAT TTCATGAAGT 24060
   AGTTAAGCAT CTGAAGAAGC ATGGAATTAG TGACAAGAAA TGATGCGGAA AAGATATCCA 24120
   GATCCAATCA AGAAGGGCCT TGTTGGCATT CTATGGAGTC TGGACTTTGG CTTCTGGGTC 24180
   ACAAGTTCTC AGATGGGGTT TTCATATCTA TTATTAGACC TACTATGTAC TGGTCCAGTG 24240
   GAAGGGAAAG GGGTTGTCTT ACTGCTAGTG GAGTAGGAAT TGGGTATGGA CCACAGCTTG 24300
   TCTTGTTTCC AAGTATTCCC TAAGAAATCT GGTCTGCTGA TGGGAGATCT ATTTATGGAA 24360
   ATGTCTTTTT CCCTCAGGAA TTTTATGTCG GAAACAGCTG TCATAGGTGA GGAGGAACTG 24420
   GTAAAAGTAC TTAATAGGAG AGTGTCATGG TCAGATTGGT GTTTTGGAAA AGTCAGCCAG 24480
   GGCAGATTGG AGAGGTCCAT ATTGGAGGCA GGAAGACTTA AGAGACTATT GCAAAGGTGA 24540
   AGACAAAAGA CGATAGGGAC TTGCACTTTA ATTCCAGCCC TTAGAAGTAG TAGAAGGTCA 24600
   GAAATGAGAA TATGCATTAC AGAGATAGTT AGTTGCTATA TCATTAGGAC TTGGTGATAG 24660
   ATTGGATGAG GATGCGGTTG GGTGAGGCAA AGAGGAGAGT CCACATTCCT GGTCTGGGTA 24720
   GTAACAAAGA ATCTAGCAAG AGGGCTTGTG GGGAAAGATG CTGAGTTACG TAGCAAGTGC 24780
   ATCTGCTTTA TCCTTGTAAT GAATGGGGCT AAAGGTGTAA ACCAAAGAGT CATCAGCATT 24840
   TGGAGGGTAG AATAAATCAT CAGATAACTC AGGAAGAAGG AGCAGAAGAA TTACTGATAC 24900
   TCCCTGGAAG GAAAACCGGA AGTAAATGGG AGAAACTTGC TCAAGTGGAC AAAGTTTAAC 24960
   AGACATGAAG CATGAATTC                                             24979
(2) INFORMATION FOR SEQ ID NO:4:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (xi) SEQUENCE DESCRIPTION: SEQ ID NO:4:
   Met Ser Arg Lys Ser Tyr Lys His
   1 5
(2) INFORMATION FOR SEQ ID NO:5:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (xi) SEQUENCE DESCRIPTION: SEQ ID NO:5:
   Met Ser Ser Val Lys Phe Val Ser Thr Thr Tyr Ser Gly Val Thr Arg
   1 5 10 15